# EUROPEAN PATENT APPLICATION

(11) **EP 4 506 004 A1**
(43) Date of publication of application: **12.02.2025**
(21) Application number: 23778439.2
(22) Date of filing: 31.03.2023
(51) Int. Cl.: A61K 31/4985, A61K 39/395, A61P 35/00, A61P 35/02

(54) **PHARMACEUTICAL COMPOSITION, USE THEREOF, AND METHOD FOR TREATING CANCER**

(30) Priority: 01.04.2022 CN 202210371222
(71) Applicant: Genfleet Therapeutics (Shanghai) Inc., Shanghai 201203 (CN); Zhejiang Genfleet Therapeutics Co., Ltd., Shaoxing Binhai New City Shaoxing, Zhejiang 312000 (CN)
(72) Inventor: TANG, Lili, Shanghai 201203 (CN); YU, Xiang, Shanghai 201203 (CN); ZHANG, Jingyang, Shanghai 201203 (CN); HU, Huizhong, Shanghai 201203 (CN); ZHOU, Fusheng, Shanghai 201203 (CN); WANG, Yu, Shanghai 201203 (CN); LAN, Jiong, Shanghai 201203 (CN); LU, Qiang, Shanghai 201203 (CN)
(74) Representative: Huang, Liwei
(86) International application number: PCT/CN2023/085402
(87) International publication number: WO 2023/186075

(57) **Abstract**

Disclosed are a pharmaceutical composition or kit comprising a therapeutically effective amount of a KRAS inhibitor and a therapeutically effective amount of an additional anti-cancer therapeutic agent, and use of a KRAS inhibitor and at least one additional anti-cancer therapeutic agent in preparing a medicament for treating a cancer. The KRAS inhibitor and the additional anti-cancer therapeutic agent have a good synergistic effect in combined use in tumor cells.

## Description

### TECHNICAL FIELD

The present invention relates to the technical field of pharmaceuticals, and particularly, to a combined pharmaceutical composition containing a KRAS inhibitor.

### BACKGROUND

Cancer is one of the major diseases threatening the human health worldwide. Lung cancer is the most prevalent cancer worldwide. In China, lung cancer ranks first among all cancers in morbidity, and possesses the highest morbidity and mortality in China. According to the data published by the American Cancer Society in 2016, about 1.8 million people develop lung cancer annually worldwide, of which nearly 80% are non-small cell lung cancers (NSCLCs).

RAS is a group of closely related monomeric globulin (with a molecular weight of 21 kDa) having 188-189 amino acids and capable of binding to guanosine diphosphate (GDP) or guanosine triphosphate (GTP). RAS subfamily members include HRAS, KRAS, and NRAS. RAS functions as a molecular switch, being in the dormant or off state and "inactive" when it contains bound GDP. When cells are exposed to certain growth-promoting stimuli, RAS is induced to convert the bound GDP to GTP. When binding to GTP, RAS is "turned on" and can interact with and activate other downstream target proteins. The RAS protein itself has a very low intrinsic ability to hydrolyze GTP and restore GDP (thereby switching itself to the off state). An exogenous protein, GTPase-activating protein (GAP), is required to return RAS to the off state. The interaction between GAP and RAS greatly accelerates the conversion of GTP to GDP. Any mutation in RAS that affects the interaction of RAS with GAP and the ability to convert GTP to GDP will result in an extended activated state of the protein, thereby extending the cell signaling process and thus resulting in continued cell growth and division. Since this signaling causes cell growth and division, overactive RAS signaling can ultimately result in a cancer. RAS gene mutations are identified in about 32% of lung cancers, and the mutation in any one of the three major subtypes of the RAS gene (HRAS, NRAS, or KRAS) may lead to the development of tumors in humans. It has been reported that the highest mutation frequency among RAS genes was observed in the KRAS gene, and KRAS mutation was detected in 25-30% of tumors. In comparison, the rates of oncogenic mutations in NRAS and HRAS family members are much lower (8% and 3%, respectively). The most common KRAS mutations are found at residues G12 and G13 in the P-loop and residue Q61. The G12C mutation is a frequent mutation in the KRAS gene (a glycine-12 mutation to cysteine). This mutation has been found in about 13% of cancers, about 43% of lung cancers, and almost 100% of MYH-related polyposis (familial colon cancer syndrome). Therefore, the development of inhibitors for selectively inhibiting KRAS mutations is a prospective orientation. Although KRAS inhibitors as monotherapy have shown efficacy in certain cancers, there is still a need for more effective therapies against cancers.

### SUMMARY

The present invention is intended to provide a pharmaceutical composition or a kit comprising a therapeutically effective amount of a KRAS inhibitor and a therapeutically effective amount of an additional anti-cancer therapeutic agent, and use of a KRAS inhibitor and at least one additional anti-cancer therapeutic agent in preparing a medicament for treating a cancer. The KRAS inhibitor and the additional anti-cancer therapeutic agent have a good synergistic effect in combined use in tumor cells.

In one aspect of the present invention, provided is a pharmaceutical composition or kit, comprising a therapeutically effective amount of a KRAS inhibitor and a therapeutically effective amount of at least one additional anti-cancer therapeutic agent, as well as a pharmaceutically acceptable carrier.

The KRAS inhibitor comprises the compound of formula (I), or a pharmaceutically acceptable salt, a stereoisomer, a solvate, or a prodrug thereof; wherein R is CH₃ or CD₃;

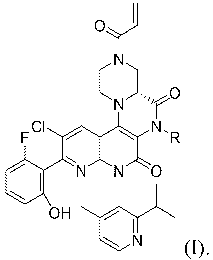

In one embodiment, the additional anti-cancer therapeutic agent is selected from a PD-1 pathway inhibitor, a PD-L1 pathway inhibitor, an EGFR-targeting agent, a CDK inhibitor, and a PI3K inhibitor.

In one embodiment, the PD-1 pathway inhibitor is selected from a PD-1 antagonist, a PD-1-binding antagonist, a small molecule PD-1 antagonist, a PD-1 inhibitor, and an anti-PD-1 bioproduct.

In one embodiment, the PD-L1 pathway inhibitor is selected from a PD-L1 antagonist, a PD-L1-binding antagonist, a small molecule PD-L1 antagonist, a PD-L1 inhibitor, and an anti-PD-L1 bioproduct.

In one embodiment, the EGFR-targeting agent is selected from an EGFR inhibitor and an EGFR antibody or an antigen-binding fragment thereof.

In one embodiment, the CDK inhibitor is selected from a CDK4/6 inhibitor, a CDK2/4/6 inhibitor, a CDK7 inhibitor, a CDK8 inhibitor, a CDK9 inhibitor, a CDK10 inhibitor, and a CDK11 inhibitor.

In one embodiment, the PI3K inhibitor is selected from a PI3Kγ inhibitor, a PI3Kδ inhibitor, a PI3Kβ inhibitor, a PI3Kα inhibitor, and a pan-PI3K inhibitor.

In one preferred embodiment, the pharmaceutical composition or kit comprises the compound of formula (I) and the PD-1 inhibitor.

In one preferred embodiment, the pharmaceutical composition or kit comprises the compound of formula (I) and the PD-L1 inhibitor.

In one preferred embodiment, the pharmaceutical composition or kit comprises the compound of formula (I) and the EGFR inhibitor.

In one preferred embodiment, the pharmaceutical composition or kit comprises the compound of formula (I) and the EGFR antibody or the antigen-binding fragment thereof.

In one preferred embodiment, the pharmaceutical composition or kit comprises the compound of formula (I) and the CDK7 inhibitor.

In one preferred embodiment, the pharmaceutical composition or kit comprises the compound of formula (I) and the PI3K inhibitor.

In one embodiment, the PD-1 inhibitor is selected from AMG404, pembrolizumab, nivolumab (MDX 1106), pembrolizumab (MK-3475), pidilizumab (CT-011), cemiplimab (REGN2810), tislelizumab (BGB-A317), spartalizumab (PDR001), RN888, mAb15, MEDI-0680 (AMP-514), BGB-108, spartalizumab (AGEN-2034), IBI-308, mDX-400, SHR-1210, PF-06801591, PDR-001, GB-226 and STI-1110, and a biosimilar, a biobetter and a bioequivalent of the inhibitors.

In one embodiment, the PD-L1 inhibitor is selected from BMS-936559 (MDX-1105), AMP-714, atezolizumab (MPDL3280A), durvalumab (MEDI4736), avelumab, avelumab, ALN-PDL, TSR-042, KD-033, CA-170, STI-1014 and KY-1003, and a biosimilar, a biobetter and a bioequivalent of the inhibitors.

In one embodiment, the EGFR inhibitor is selected from afatinib, erlotinib, lapatinib, gefitinib, osimertinib, icotinib, pyrotinib, and olmutinib.

In one embodiment, the EGFR antibody or the antigen-binding fragment thereof is selected from cetuximab, nimotuzumab, panitumumab, zalutumumab, matuzumab, and necitumumab, more preferably, cetuximab.

In one embodiment, the CDK7 inhibitor is selected from SY-5609, SY-1365, CT-7001, and BTX-A51.

In one preferred embodiment, the pharmaceutical composition or kit comprises the compound of formula (I) and SY-5609.

In one embodiment, the PI3K inhibitor is selected from BYL719, GDC0941, AMG511, LY294002, BEZ235, BKM120, and GSK-2636771.

In one preferred embodiment, the pharmaceutical composition or kit comprises the compound of formula (I) and BYL719.

In one preferred embodiment, the pharmaceutical composition or kit comprises the compound of formula (I) and GDC0941.

In one embodiment, the cancer is a KRAS G12C mutant cancer.

In one embodiment, the cancer is a solid tumor or a hematological tumor.

In one embodiment, the cancer is pancreatic ductal carcinoma, colorectal cancer, multiple myeloma, lung cancer, cutaneous melanoma, uterine corpus endometrioid carcinoma, uterine carcinosarcoma, thyroid cancer, acute myeloid leukemia, bladder urothelial carcinoma, gastric cancer, cervical cancer, head and neck squamous cell carcinoma, diffuse large B cell lymphoma, esophageal cancer, chronic lymphocytic leukemia, squamous cell lung cancer, small cell lung cancer, papillary renal cell carcinoma, adenoid cystic carcinoma, chromophobe renal cell carcinoma, liver cancer, invasive breast carcinoma, cervical squamous cell carcinoma, ovarian serous adenocarcinoma, adrenocortical carcinoma, prostate cancer, neuroblastoma, brain low-grade glioma, glioblastoma, medulloblastoma, esophageal squamous cell carcinoma, clear cell renal cell carcinoma, osteosarcoma, ovarian small cell carcinoma, rhabdomyoid tumor, sarcoma, small bowel neuroendocrine tumor, or T cell prolymphocytic leukemia.

In one embodiment, the cancer is lung adenocarcinoma, colon cancer, rectal cancer, or lung cancer.

In one embodiment, the cancer is lung adenocarcinoma, rectal adenocarcinoma, or lung cancer.

In one embodiment, the lung cancer is small cell lung cancer or non-small cell lung cancer.

In one embodiment, the compound of formula (I) is selected from the following group:

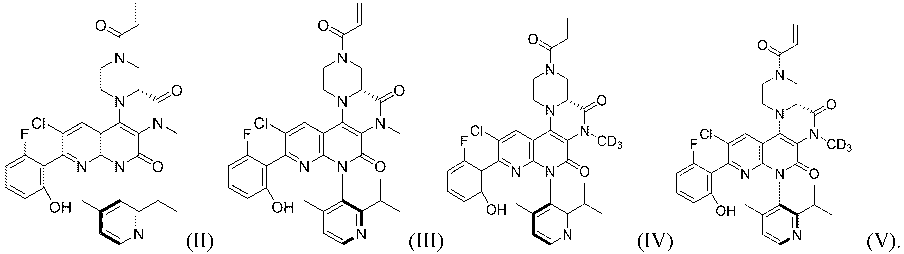

In one embodiment, the KRAS inhibitor is the compound of formula (II) or a pharmaceutically acceptable salt thereof.

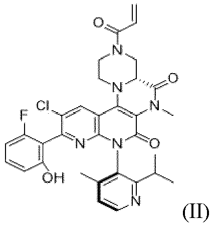

In one embodiment, the pharmaceutical composition or kit comprises the compound of formula (II) and the PD-1 pathway inhibitor.

In one embodiment, the pharmaceutical composition or kit comprises the compound of formula (II) and the PD-L1 pathway inhibitor.

In one embodiment, the pharmaceutical composition or kit comprises the compound of formula (II) and the EGFR-targeting agent.

In one preferred embodiment, the pharmaceutical composition or kit comprises the compound of formula (II) and cetuximab.

In one embodiment, the pharmaceutical composition or kit comprises the compound of formula (II) and the CDK7 inhibitor.

In one preferred embodiment, the pharmaceutical composition or kit comprises the compound of formula (II) and SY-5609.

In one embodiment, the pharmaceutical composition or kit comprises the compound of formula (II) and the PI3K inhibitor.

In one preferred embodiment, the pharmaceutical composition or kit comprises the compound of formula (II) and BYL719.

In one preferred embodiment, the pharmaceutical composition or kit comprises the compound of formula (II) and GDC0941.

In one embodiment, the pharmaceutical composition or kit comprises 0.1-1000 nM of the KRAS inhibitor and 1-10000 nM of the additional anti-cancer therapeutic agent.

In one embodiment, the pharmaceutical composition or kit comprises 0.32-1000 nM of the KRAS inhibitor.

In one embodiment, the pharmaceutical composition or kit comprises 3-1000 nM of the KRAS inhibitor.

In one embodiment, the pharmaceutical composition or kit comprises 3.17-1000 nM of the KRAS inhibitor.

In one embodiment, the pharmaceutical composition or kit comprises 10-1000 nM of the KRAS inhibitor.

In one embodiment, the pharmaceutical composition or kit comprises 0.1-100 nM of the KRAS inhibitor.

In one embodiment, the pharmaceutical composition or kit comprises 3.17-1000 nM of the additional anti-cancer therapeutic agent.

In one embodiment, the pharmaceutical composition or kit comprises 31.74-10000 nM of the additional anti-cancer therapeutic agent.

In one embodiment, the pharmaceutical composition or kit comprises 100-10000 nM of the additional anti-cancer therapeutic agent.

In one embodiment, the pharmaceutical composition or kit comprises 316.91-10000 nM of the additional anti-cancer therapeutic agent.

In one embodiment, the pharmaceutical composition or kit comprises 10 mg/mL of the compound of formula (II) and 1 mg/mL of cetuximab.

In one embodiment, the pharmaceutical composition or kit comprises 1-3 mg/kg of the compound of formula (II) and 10 mg/kg of the PD-1 antibody.

In one embodiment, the pharmaceutical composition or kit comprises a pharmaceutical dose equivalent to 10 mpk of the compound of formula (II) and 1 mpk of cetuximab.

In one embodiment, the pharmaceutical composition or kit comprises a pharmaceutical dose equivalent to 1-3 mpk of the compound of formula (II) and 10 mpk of the PD-1 antibody.

In one embodiment, the pharmaceutical composition or kit comprises 0.1-100 nM of the compound of formula (II) and 1-1000 nM of SY-5609.

In one embodiment, the pharmaceutical composition or kit comprises 0.1-100 nM of the compound of formula (II) and 3.17-1000 nM of SY-5609.

In one embodiment, the pharmaceutical composition or kit comprises 1-1000 nM of the compound of formula (II) and 100-10000 nM of BYL719.

In one embodiment, the pharmaceutical composition or kit comprises 3-1000 nM of the compound of formula (II) and 100-10000 nM of BYL719.

In one embodiment, the pharmaceutical composition or kit comprises 10-1000 nM of the compound of formula (II) and 100-10000 nM of BYL719.

In one embodiment, the pharmaceutical composition or kit comprises 0.1-1000 nM of the compound of formula (II) and 10-10000 nM of GDC0941.

In one embodiment, the pharmaceutical composition or kit comprises 3.17-1000 nM of the compound of formula (II) and 31.74-10000 nM of GDC0941.

In one embodiment, the pharmaceutical composition or kit comprises 3.17-1000 nM of the compound of formula (II) and 100-10000 nM of GDC0941.

In one embodiment, the pharmaceutical composition or kit comprises 10-1000 nM of the compound of formula (II) and 31.74-10000 nM of GDC0941.

In one embodiment, the pharmaceutical composition or kit comprises 0.32-1000 nM of the compound of formula (II) and 316.91-10000 nM of GDC0941.

In one embodiment, the KRAS inhibitor and the additional anti-cancer therapeutic agent are present in the pharmaceutical composition or kit in a mass ratio of 0.00001-1000.

In one embodiment, the pharmaceutical composition or kit comprises the compound of formula (II) and cetuximab in a mass ratio of (1-10): 1, e.g., 10:1.

In one embodiment, the pharmaceutical composition or kit comprises the compound of formula (II) and the PD-1 antibody in a mass ratio of (1-10):10, e.g., 1:10 or 3:10.

In one embodiment, the pharmaceutical composition or kit comprises the compound of formula (II) and SY-5609 in a mass ratio of 0.0001-31.6.

In one embodiment, the pharmaceutical composition or kit comprises the compound of formula (II) and BYL719 in a mass ratio of 0.0003-10, e.g., 0.001-10 or 0.0003-10.

In one embodiment, the pharmaceutical composition or kit comprises the compound of formula (II) and GDC0941 in a mass ratio of 0.000032-35, e.g., 0.001-31.5, 0.0003-10, 0.0003-31.5, or 0.00003-3.15.

In one embodiment, the pharmaceutical composition or kit comprises a pharmaceutical dose of the compound of formula (II) equivalent to 0.01-5000 mg/day, preferably 1-1500 mg/day, optionally 10 mg/day, 50 mg/day, 100 mg/day, 150 mg/day, 200 mg/day, 250 mg/day, 300 mg/day, 350 mg/day, 400 mg/day, 400 mg/day, 500 mg/day, 550 mg/day, 600 mg/day, 650 mg/day, 700 mg/day, 750 mg/day, 800 mg/day, 8500 mg/day, 900 mg/day, 950 mg/day, 1000 mg/day, 1200 mg/day, 1250 mg/day, 1300 mg/day, 1400 mg/day, or 1500 mg/day.

In one embodiment, the pharmaceutical composition or kit comprises a pharmaceutical dose of the additional anti-cancer therapeutic agent equivalent to 0.01-1000 mg/day, preferably 1-500 mg/day, optionally 10 mg/day, 50 mg/day, 100 mg/day, 150 mg/day, 200 mg/day, 250 mg/day, 300 mg/day, 350 mg/day, 400 mg/day, 400 mg/day, 500 mg/day, 550 mg/day, 600 mg/day, 650 mg/day, 700 mg/day, 750 mg/day, 800 mg/day, 8500 mg/day, 900 mg/day, 950 mg/day, or 1000 mg/day.

In one embodiment, the pharmaceutical composition or kit comprises 1-10000 mg of the KRAS inhibitor and 1-10000 mg of the additional anti-cancer therapeutic agent.

In one embodiment, the pharmaceutical composition or kit comprises 1-10000 mg of the compound of formula (II) and 1-10000 mg of cetuximab.

In one embodiment, the pharmaceutical composition or kit comprises 1-10000 mg of the compound of formula (II) and 1-10000 mg of SY-5609.

In one embodiment, the pharmaceutical composition or kit comprises 1-10000 mg of the compound of formula (II) and 1-10000 mg of BYL719.

In one embodiment, the pharmaceutical composition or kit comprises 1-10000 mg of the compound of formula (II) and 1-10000 mg of GDC0941.

In another aspect of the present invention, provided is a method for treating a cancer, comprising: administering to a subject in need the KRAS inhibitor and the additional anti-cancer therapeutic agent described above, wherein a therapeutically effective amount of the KRAS inhibitor and a therapeutically effective amount of the additional anti-cancer therapeutic agents may be administered simultaneously, separately, or sequentially.

In one embodiment, the method further comprises: administering to the subject an additional therapy selected from one or more of a radiation therapy, a surgery, a chemotherapy, a gene therapy, a DNA therapy, a viral therapy, an RNA therapy, an immunotherapy, bone marrow transplantation, a nano-therapy, a monoclonal antibody therapy, and a phototherapy. The additional therapy may be in the form of an adjuvant therapy or a neoadjuvant therapy.

In another aspect of the present invention, provided is use of the pharmaceutical composition described above in preparing a medicament for treating a cancer. A therapeutically effective amount of the KRAS inhibitor and a therapeutically effective amount of the additional anti-cancer therapeutic agent may be administered simultaneously, separately, or sequentially.

In one embodiment, the cancer in the use described above is a solid tumor or a hematological tumor.

In one embodiment, the cancer in the use described above is pancreatic ductal carcinoma, colorectal cancer, multiple myeloma, lung cancer, cutaneous melanoma, uterine corpus endometrioid carcinoma, uterine carcinosarcoma, thyroid cancer, acute myeloid leukemia, bladder urothelial carcinoma, gastric cancer, cervical cancer, head and neck squamous cell carcinoma, diffuse large B cell lymphoma, esophageal cancer, chronic lymphocytic leukemia, squamous cell lung cancer, small cell lung cancer, papillary renal cell carcinoma, adenoid cystic carcinoma, chromophobe renal cell carcinoma, liver cancer, invasive breast carcinoma, cervical squamous cell carcinoma, ovarian serous adenocarcinoma, adrenocortical carcinoma, prostate cancer, neuroblastoma, brain low-grade glioma, glioblastoma, medulloblastoma, esophageal squamous cell carcinoma, clear cell renal cell carcinoma, osteosarcoma, ovarian small cell carcinoma, rhabdomyoid tumor, sarcoma, small bowel neuroendocrine tumor, or T cell prolymphocytic leukemia.

In one embodiment, the cancer in the use described above is lung adenocarcinoma, colon cancer, rectal cancer, or lung cancer.

In one embodiment, the cancer in the use described above is lung adenocarcinoma, rectal adenocarcinoma, or lung cancer.

In one embodiment, the cancer in the use described above is a KRAS G12C mutant cancer.

In one embodiment, the cancer in the use described above is small cell lung cancer or non-small cell lung cancer.

In another aspect of the present invention, provided is use of a therapeutically effective amount of a KRAS inhibitor and a therapeutically effective amount of at least one additional anti-cancer therapeutic agent in preparing a medicament, a pharmaceutical combination, or a kit for treating a cancer, wherein the KRAS inhibitor is selected from one or more of the compound of formula (I) described above, the compounds of formula (II)-formula (V) described above, or a pharmaceutically acceptable salt, a stereoisomer, a solvate, or a prodrug thereof. The KRAS inhibitor, the additional anti-cancer therapeutic agent, and the cancer in the use are as defined in the aforementioned pharmaceutical composition or kit.

Specifically, in one aspect, the present invention provides use of a therapeutically effective amount of the compound of formula (I) and a therapeutically effective amount of at least one additional anti-cancer therapeutic agent in preparing a medicament, a pharmaceutical combination, or a kit for treating a cancer.

In one aspect, the present invention provides use of a therapeutically effective amount of the compound of formula (II) and a therapeutically effective amount of at least one additional anti-cancer therapeutic agent in preparing a medicament, a pharmaceutical combination, or a kit for treating a cancer.

In one aspect, the present invention provides use of a therapeutically effective amount of the compound of formula (I) and a therapeutically effective amount of at least one additional anti-cancer therapeutic agent in preparing a medicament, a pharmaceutical combination, or a kit for treating lung cancer.

In one aspect, the present invention provides use of a therapeutically effective amount of the compound of formula (I) and a therapeutically effective amount of at least one additional anti-cancer therapeutic agent in preparing a medicament, a pharmaceutical combination, or a kit for treating small cell lung cancer or non-small cell lung cancer.

In one aspect, the present invention provides use of a therapeutically effective amount of the compound of formula (II) and a therapeutically effective amount of at least one additional anti-cancer therapeutic agent in preparing a medicament, a pharmaceutical combination, or a kit for treating lung cancer.

In one aspect, the present invention provides use of a therapeutically effective amount of the compound of formula (II) and a therapeutically effective amount of at least one additional anti-cancer therapeutic agent in preparing a medicament, a pharmaceutical combination, or a kit for treating small cell lung cancer or non-small cell lung cancer.

In one aspect, the present invention provides use of a therapeutically effective amount of the compound of formula (II) and a therapeutically effective amount of the PD-1 inhibitor in preparing a medicament, a pharmaceutical combination, or a kit for treating small cell lung cancer, non-small cell lung cancer, lung adenocarcinoma, colon cancer, or rectal adenocarcinoma.

In one aspect, the present invention provides use of a therapeutically effective amount of the compound of formula (II) and a therapeutically effective amount of the PD-L1 inhibitor in preparing a medicament, a pharmaceutical combination, or a kit for treating small cell lung cancer, non-small cell lung cancer, lung adenocarcinoma, colon cancer, or rectal adenocarcinoma.

In one aspect, the present invention provides use of a therapeutically effective amount of the compound of formula (II) and a therapeutically effective amount of the EGFR inhibitor in preparing a medicament, a pharmaceutical combination, or a kit for treating small cell lung cancer, non-small cell lung cancer, lung adenocarcinoma, colon cancer, or rectal adenocarcinoma.

In one aspect, the present invention provides use of a therapeutically effective amount of the compound of formula (II) and a therapeutically effective amount of the EGFR antibody or the antigen-binding fragment thereof in preparing a medicament, a pharmaceutical combination, or a kit for treating small cell lung cancer, non-small cell lung cancer, lung adenocarcinoma, colon cancer, or rectal adenocarcinoma.

In one aspect, the present invention provides use of a therapeutically effective amount of the compound of formula (II) and a therapeutically effective amount of the CDK7 inhibitor in preparing a medicament, a pharmaceutical combination, or a kit for treating small cell lung cancer, non-small cell lung cancer, lung adenocarcinoma, colon cancer, or rectal adenocarcinoma.

In one aspect, the present invention provides use of a therapeutically effective amount of the compound of formula (II) and a therapeutically effective amount of the PI3K inhibitor in preparing a medicament, a pharmaceutical combination, or a kit for treating small cell lung cancer, non-small cell lung cancer, lung adenocarcinoma, colon cancer, or rectal adenocarcinoma.

### BRIEF DESCRIPTION OF THE DRAWINGS

FIG. 1 is a matrix illustrating the inhibited growth of tumor cells LU99 by the compound of formula (II) in combination with BYL719 according to Example 1.
FIGs. 2A-2D illustrate the mean synergic values calculated using the four statistical models according to Example 1.
FIG. 3 is a matrix illustrating the inhibited growth of tumor cells SW837 by the compound of formula (II) in combination with BYL719 according to Example 2.
FIGs. 4A-4D illustrate the mean synergic values calculated using the four statistical models according to Example 2.
FIG. 5 is a matrix illustrating the inhibited growth of tumor cells LU99 by the compound of formula (II) in combination with GDC0941 according to Example 3.
FIGs. 6A-6D illustrate the mean synergic values calculated using the four statistical models according to Example 4.
FIG. 7 is a matrix illustrating the inhibited growth of tumor cells SW837 by the compound of formula (II) in combination with GDC0941 according to Example 4.
FIGs. 8A-8D illustrate the mean synergic values calculated using the four statistical models according to Example 4.
FIG. 9 is a matrix illustrating the inhibited growth of tumor cells SW1463 by the compound of formula (II) in combination with GDC0941 according to Example 5.
FIGs. 10A-10D illustrate the mean synergic values calculated using the four statistical models according to Example 5.
FIG. 11 is a matrix illustrating the inhibited growth of tumor cells SW1573 by the compound of formula (II) in combination with GDC0941 according to Example 6.
FIGs. 12A-12D illustrate the mean synergic values calculated using the four statistical models according to Example 6.
FIG. 13 is a matrix illustrating the inhibited growth of tumor cells NCI-H2122 by the compound of formula (II) in combination with SY-5609 according to Example 7.
FIGs. 14A-14D illustrate the mean synergic values calculated using the four statistical models according to Example 7.
FIG. 15 illustrates the growth curves (mean ± standard error) of tumors in tumor-bearing mice in each group according to Example 8.
FIG. 16 illustrates the growth curves of tumors in individual tumor-bearing mice in each group according to Example 8.
FIG. 17 illustrates the weight (mean ± standard error) of tumors in tumor-bearing mice in each group according to Example 8.
FIG. 18 illustrates the relative change in body weight (mean ± standard error) of tumor-bearing mice according to Example 8.
FIG. 19 illustrates the growth curves (mean ± standard error) of tumors in tumor-bearing mice in each group according to Example 9.
FIG. 20 illustrates the growth curves of tumors in individual tumor-bearing mice in each group according to Example 9.
FIG. 21 illustrates the weight (mean ± standard error) of tumors in tumor-bearing mice in each group according to Example 9.
FIG. 22 illustrates the relative change in body weight (mean ± standard error) of tumor-bearing mice according to Example 9.

### DETAILED DESCRIPTION

### I. Terminology and Definitions

Unless otherwise stated, the following terms and phrases used herein are intended to have the following meanings. A specific term or phrase, unless otherwise specifically defined, should not be considered uncertain or unclear, but interpreted according to its ordinary meaning. When referring to a trade name, it is intended to refer to its corresponding commercial product or its active ingredient.

As used herein, unless otherwise stated, the terms "comprise", "include", "have", and "contain", including grammatical equivalents thereof, should generally be interpreted as open-ended and non-limiting, for example, without excluding other unrecited elements or procedures.

As used herein, the term "inhibit" is used relative to a control. Those skilled in the art will readily determine the appropriate control for each experiment. For example, a decreased response in a subject or cell treated with a compound is compared with a response in a subject or cell not treated with the compound. The disclosure of all ranges in the present invention should be construed as disclosing all sub-ranges and all point values within the range. For example: the disclosure of 1-1000 should be construed to also disclose the ranges of 1-200, 200-300, and the like, as well as the point values of 200, 300, 400, 500, 600, 700, 800, 900, 1000, and the like.

The term "pharmaceutically acceptable" refers to those compounds, materials, compositions, and/or dosage forms that are, within the scope of sound medical judgment, suitable for use in contact with the tissues of humans and animals without excessive toxicity, irritation, allergic response, or other problems or complications, and commensurate with a reasonable benefit/risk ratio.

The compound may be present in the pharmaceutical composition as a pharmaceutically acceptable salt. The term "pharmaceutically acceptable salt" refers to a salt of the compound of the present invention, which is prepared from the compound of the present invention having specific substituents and a relatively nontoxic acid or base. When the compound of the present invention contains a relatively acidic functional group, a base addition salt can be given by contacting the neutral form of such a compound with a sufficient amount of a base in a pure solution or a suitable inert solvent. Pharmaceutically acceptable base addition salts include sodium, potassium, calcium, ammonium, organic amine, or magnesium salts, or similar salts. When the compound of the present invention contains a relatively basic functional group, an acid addition salt can be given by contacting the neutral form of such a compound with a sufficient amount of an acid in a pure solution or a suitable inert solvent. Examples of pharmaceutically acceptable acid addition salts include salts derived from inorganic acids, such as hydrochloric acid, hydrobromic acid, nitric acid, carbonic acid, bicarbonate radical, phosphoric acid, monohydrogen phosphate, dihydrogen phosphate, sulfuric acid, hydrogen sulfate, hydroiodic acid, and phosphorous acid; and salts derived from organic acids, such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid. Also included are salts of amino acids (e.g., arginine) and salts of organic acids such as glucuronic acid. Certain specific compounds of the present invention contain both basic and acidic functional groups that allow the compounds to be converted to either base or acid addition salts.

The pharmaceutically acceptable salt of the present invention can be synthesized from the parent compound containing an acid radical or a basic group by conventional chemical methods. In general, such salts are prepared by subjecting the compounds in a free acid or base form to a reaction with a stoichiometric amount of appropriate base or acid in water or an organic solvent or a mixture thereof.

In addition to salt forms, the compounds provided herein also be present in prodrug forms. Prodrugs of the compounds described herein may readily undergo chemical changes under physiological conditions and thus be converted to the compounds of the present invention. In addition, prodrugs can be converted to the compounds of the present invention *in vivo* by chemical or biochemical methods. For example, when placed in a transdermal patch reservoir having the suitable enzyme or chemical reagent, the prodrug can be slowly converted to the compound of the present invention.

Certain compounds of the present invention may be present in non-solvate forms or solvate forms, including a hydrate form. In general, the solvate forms are equivalent to non-solvate forms and are included within the scope of the present invention. The solvate forms are generally equivalent to non-solvate forms and should be included within the scope of the present invention. Certain compounds of the present invention may be present in polymorphic or amorphous forms. Generally, all physical forms are equivalent for the use contemplated by the present invention and should be included within the scope of the present invention.

The compound of the present invention may be present in the form of a specific geometric isomer or stereoisomer. All such compounds are contemplated by the present invention, including *cis* and *trans* isomers, (-)- and (+)-enantiomers, (R)- and (S)-enantiomers, diastereoisomers, (D)-isomers, (L)-isomers, and racemic mixtures and other mixtures thereof, such as an enantiomer or diastereoisomer enriched mixture, all of which are included within the scope of the present invention.

The compound disclosed herein may be present as atropisomers, which are conformational stereoisomers present when the rotation about a single bond in a molecule is prevented or greatly slowed due to steric interactions with other parts of the molecule. The compound disclosed herein includes all atropisomers as pure individual atropisomer formulations, enriched formulations of each, or non-specific mixtures of each. The separation and isolation of isomers may be allowed if the potential barrier for the rotation about a single bond is sufficiently high and the enantiotropy between conformations is sufficiently slow. Separated or isolated isomers are suitably indicated by the well known and widely accepted symbol "M" or "P".

The term "cancer" refers to a disease characterized by the uncontrolled growth of abnormal cells. Cancer cells can spread locally or to other sites of the body through the blood circulation and lymphatic system. Examples of various cancers are described herein and include, but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer, and the like. The terms "tumor" and "cancer" are used interchangeably herein; for example, the two terms include solid and liquid, e.g., diffuse or circulating tumors. As used herein, the terms "cancer" and "tumor" include precancerous lesions as well as malignant cancers and tumors.

The term "antigen-binding fragment" refers to any portion of a full-length antibody that is less than the full length, but comprises at least a portion of the variable region of the antibody that binds to the antigen (e.g., one or more CDRs and/or one or more antibody binding sites), and thus retains the binding specificity as well as at least a portion of the specific binding capacity of the full-length antibody. Thus, an antigen-binding fragment refers to an antibody fragment that comprises an antigen-binding portion that binds to the same antigen as the antibody from which the antibody fragment is derived. The antibody fragments include antibody derivatives produced by enzyme treatment of full-length antibodies, as well as synthesized derivatives, e.g., derivatives produced by recombination. The antibodies include antibody fragments. Examples of antibody fragments include, but are not limited to, Fab, Fab', F(ab')2, single-chain Fv (scFv), Fv, dsFv, diabody, Fd, and Fd' fragments, as well as other fragments, including modified fragments. The fragment may comprise multiple chains linked together, for example, by a disulfide bond and/or by a peptide linker. The antibody fragment generally comprises at least or about 50 amino acids, and typically at least or about 200 amino acids. The antigen-binding fragment includes any antibody fragment that, when inserted into an antibody framework (e.g., by replacing the corresponding region), yields an antibody that immunospecifically binds to an antigen (i.e., with a Ka of at least or at least about 10⁷-10⁸ M⁻¹). The "functional fragment" or the "analog of an anti-GPC3 antibody" is a fragment or an analog that can prevent or substantially reduce the ability of the receptor to bind to a ligand or initiate signal transduction. As used herein, the functional fragment generally has the same meaning as the "antibody fragment" and, with respect to an antibody, may refer to a fragment that prevents or substantially reduces the ability of the receptor to bind to a ligand or initiate signal transduction, for example, Fv, Fab, F(ab')2, or the like. The "Fv" fragment consists of a dimer (VH-VL dimer) formed by the variable domain of one heavy chain and the variable domain of one light chain through non-covalent binding. In this configuration, the CDRs of the variable domains interact to define a target binding site on the surface of the VH-VL dimer, as is the case with intact antibodies. The six CDRs collectively confer target binding specificity to the intact antibody. However, even a single variable domain (or half of an Fv comprising only 3 target-specific CDRs) may still have the ability to recognize and bind to a target.

The "effective amount" or the "therapeutically effective amount" described in the present invention includes an amount sufficient to ameliorate or prevent a symptom of a disorder or a medical disorder. The effective amount also refers to an amount sufficient to allow or facilitate diagnosis. The effective amount for a particular patient or veterinary subject may vary depending on factors such as the disorder to be treated, the general health of the patient, the method, route and dose of administration, and the severity of side effects. The effective amount may be the maximum dose or administration regimen with significant side effects or toxic effects.

The terms "subject", "individual", and "patient" are used interchangeably herein and refer to a vertebrate, preferably a mammal, more preferably a human. The mammal includes, but is not limited to, a mouse, an ape, a human, a farm animal, a sport animal, and a pet.

The amount of the compound administered may depend on the subject being treated, the age, health status, sex and body weight of the subject, the type of concurrent treatment (if any), the severity of the condition, the nature of the desired effect, the method and frequency of the treatment, and the judgment of the prescriber. The administration frequency may also depend on the pharmacodynamic effects on arterial partial pressure of oxygen. However, the most preferred dose can be adjusted for the individual subject, as understood by those skilled in the art, and determinable without undue experimentation. This generally includes adjusting the standard dose (e.g., reducing the dose if the patient has a lower body weight).

The term "pharmaceutical composition" refers to a mixture consisting of one or more of the compounds or the salts thereof of the present application and a pharmaceutically acceptable carrier. The pharmaceutical composition is intended to facilitate the administration of the compounds of the present application to an organism. The pharmaceutical composition of the present invention may comprise one or more pharmaceutically acceptable salts, antioxidants, aqueous and non-aqueous carriers, and/or adjuvants, such as preservatives, wetting agents, emulsifiers, and dispersants.

The term "pharmaceutically acceptable carrier" refers to those excipients that do not have a significant irritating effect on an organism and do not impair the biological activity and properties of the active compound. Suitable excipients are well known to those skilled in the art, for example, carbohydrate, wax, water-soluble and/or water-swellable polymer, hydrophilic or hydrophobic material, gelatin, oil, solvent, water, liposome, polymer micelle, inorganic nanocarrier, or the like.

The pharmaceutical composition of the present invention may be prepared in any pharmaceutically acceptable dosage form for oral, nasal, topical (including buccal and sublingual), rectal, vaginal, and/or parenteral administration, for example, as tablets, lozenges, capsules, pills, solutions, suspensions, syrups, injections, suppositories, inhalants, or sprays.

The term "anti-PD-L1 bioproduct" refers to an antibody or an antigen-binding fragment thereof that specifically binds to PD-1.

The term "synergism" refers to the phenomenon whereby the effects of two drugs in combination are more effective than their individual effects, as opposed to the antagonism.

The term "treatment" includes prevention and treatment, for example, treating a disease mediated by the KRAS G12C mutation includes preventing and/or treating a disease mediated by the KRAS G12C mutation.

When the PD-1 pathway inhibitor, the PD-L1 pathway inhibitor, the EGFR-targeting agent, the CDK inhibitor, and the PI3K inhibitor described in the present invention are small molecule compounds, it is intended to include pharmaceutically acceptable salts of these compounds. For example, in one embodiment, the pharmaceutical composition of the present invention comprises the compound of formula (II) and GDC0941, i.e., comprises the compound of formula (II), GDC0941, and a pharmaceutically acceptable salt of GDC0941.

### II. Examples

The KRAS inhibitor disclosed in the present invention has the following structural formula (II), and is named (4aR,8R)-3-acryloyl-11-chloro-10-(2-fluoro-6-hydroxyphenyl)-8-(2-isopropyl-4-methylpyridin-3-yl)-6-methyl -2,3,4,4a,6,8-hexahydro-1H-pyrazino[1',2':4,5]pyrazino[2,3-c][1,8]naphthyridine-5,7-dione. Its preparation is described in Example 25 of International Patent Application No. PCT/CN2020/124226.

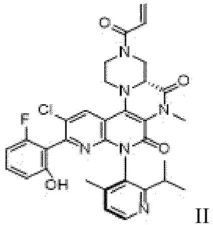

### Materials and reagents (Examples 1-7)

NCI-H2122 (ATCC, CRL-5985), LU99 (JCRB, JCRB0080), SW837 (ATCC, CRL-235), SW1463 (ATCC, CRL-234), SW1573 (ATCC, CRL-2170), L-15 (Gibco, 11415-064), RPMI1640 (Gibco, 11875-093), Trypsin-EDTA (Gibco, 25200-072), FBS (Gibco, 10099-141C), CellTiter Glo (Progema, G7573), cell counter (Count star, IC1000), cell counter (CHEMOMETEC, NC-200), and microplate reader (PerkinElmer, Envison).

### Calculation for cell survival rate in the combination groups

The cell survival rate (%) after the drug combinations was calculated by the formula: cell survival rate = (1 - (RLU of DMSO control - RLU of treatment)/(RLU of DMSO control - RLU of blank control)) × 100%. The DMSO control was a vehicle control with no drugs, and the blank control was a medium control with no drugs and vehicles.

### Interpretation of cell survival rate calculation results

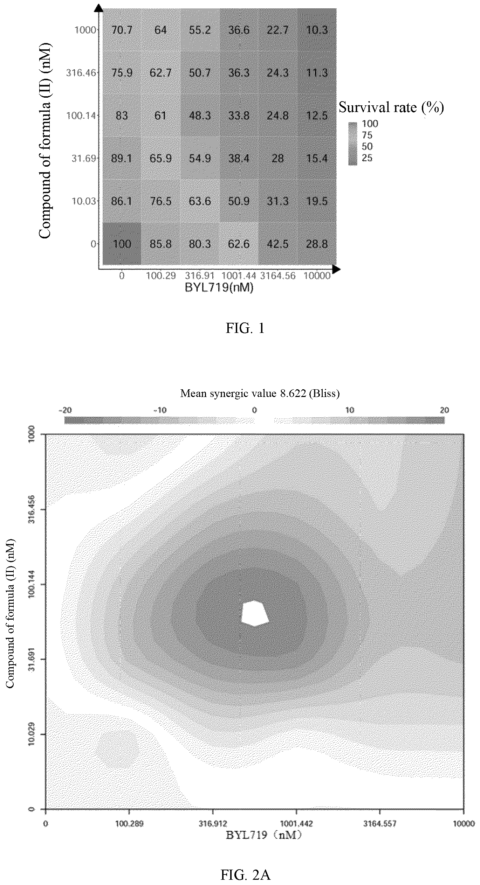

In the graphs of the cell survival rate calculation results (FIGs. 1, 3, 5, 7, 9, 11, and 13), the abscissa denotes the concentration of the KRAS inhibitor compound of formula (II) of the present application (effective concentration of the solution in the well), and the ordinate denotes the concentration of the combined therapeutic agent (effective concentration of the drug solution in the well). The value corresponding to the coordinates is the cell survival rate of the combination groups with corresponding concentrations of the two drugs. For example, in FIG. 1, when the horizontal coordinate read is 0 nM and the vertical coordinate read is 10.03 nM, the value corresponding to the coordinates is 86.1. This indicates that the cell survival rate was 86.1%. That is, when the BYL719 concentration was 0 nM, i.e., when the compound of formula (II) was administered alone, the cell survival rate was 86.1%.

### Evaluation for synergic effects

The effect of the drug combinations on the tumor cells was evaluated by statistical methods. The statistical software used was R systems, and the statistical models were Bliss, HSA, Loewe, and ZIP. A synergic value > 5 indicates a synergic effect, a synergic value > 10 indicates a significant synergic effect, a synergic value < -5 indicates an antagonistic effect, and a synergic value < -10 indicates a significant antagonistic effect. The final efficacy of the drug combinations was further judged by the synergic value.

### Example 1. Effect of combination of compound of formula (II) with BYL719 (alpelisib) on tumor cells LU99

Human non-small cell lung cancer cells LU99 were plated into a 384-well plate at 600 cells/well. After the cells adhered to the well wall (24 h), the serially diluted compound of formula (II) and BYL719 were added in duplicate for each concentration. After 120 h, the cell viability was measured by Cell Titer-Glo method. The Cell Titer-Glo reagent was added at 30 µL/well. The plate was shaken for 2 min and left to stand for 10 min. The RLU value was read on the Envision system.

The cell survival rate calculation results are shown in FIG. 1. In human non-small cell lung cancer cells LU99, for example, the survival rate of cells receiving 100 nM of the compound of formula (II) alone was 83%, the survival rate of cells receiving 1000 nM of BYL719 alone was 62.6%, and the survival rate of cells receiving the combination of 100 nM of the compound of formula (II) with 1000 nM of BYL719 was 33.8%.

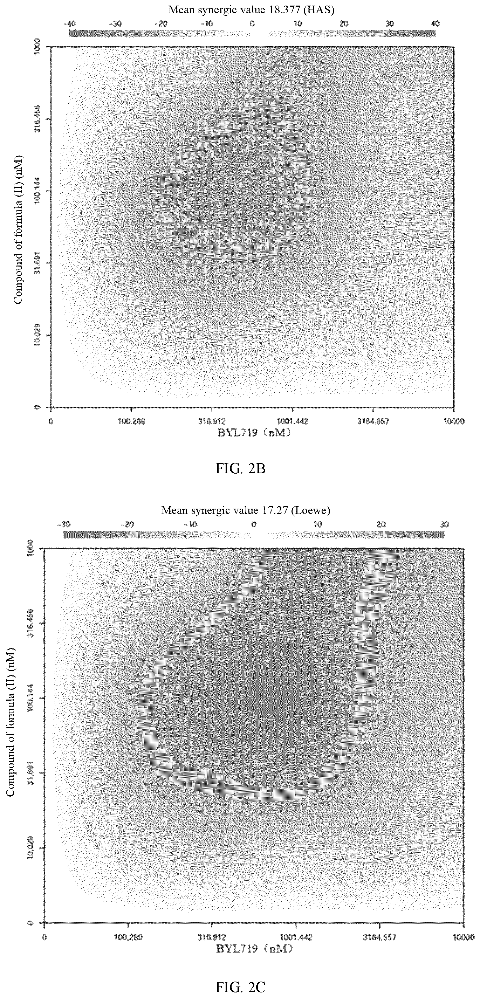

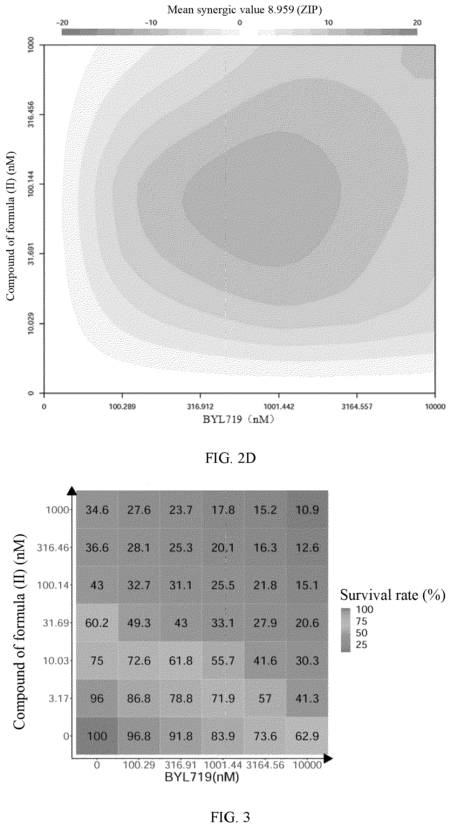

The combination effect is shown in FIG. 2. In human non-small cell lung cancer cells LU99, 10 nM-1000 nM of the compound of formula (II) was combined with 100 nM-10000 nM of BYL719. The mean synergic values calculated using the four statistical models (Bliss (FIG. 2A), HSA (FIG. 2B), Loewe (FIG. 2C), and ZIP (FIG. 2D)) were 8.622, 18.377, 17.27, and 8.959, respectively, all of which were greater than 5. The results show that the combination of the compound of formula (II) with BYL719 has a very good synergic effect in tumor cells.

### Example 2. Effect of combination of compound of formula (II) with BYL719 on tumor cells SW837

Human rectal adenocarcinoma cells SW837 were plated into a 384-well plate at 800 cells/well. After the cells adhered to the well wall (24 h), the serially diluted compound of formula (II) and BYL719 were added in duplicate for each concentration. After 120 h, the cell viability was measured by Cell Titer-Glo method. The Cell Titer-Glo reagent was added at 30 µL/well. The plate was shaken for 2 min and left to stand for 10 min. The RLU value was read on the Envision system.

The cell survival rate calculation results are shown in FIG. 3. In human rectal adenocarcinoma cells SW837, for example, the survival rate of cells receiving 31.69 nM of the compound of formula (II) alone was 60.2%, the survival rate of cells receiving 10000 nM of BYL719 alone was 62.9%, and the survival rate of cells receiving the combination of 31.69 nM of the compound of formula (II) with 10000 nM of BYL719 was 20.6%.

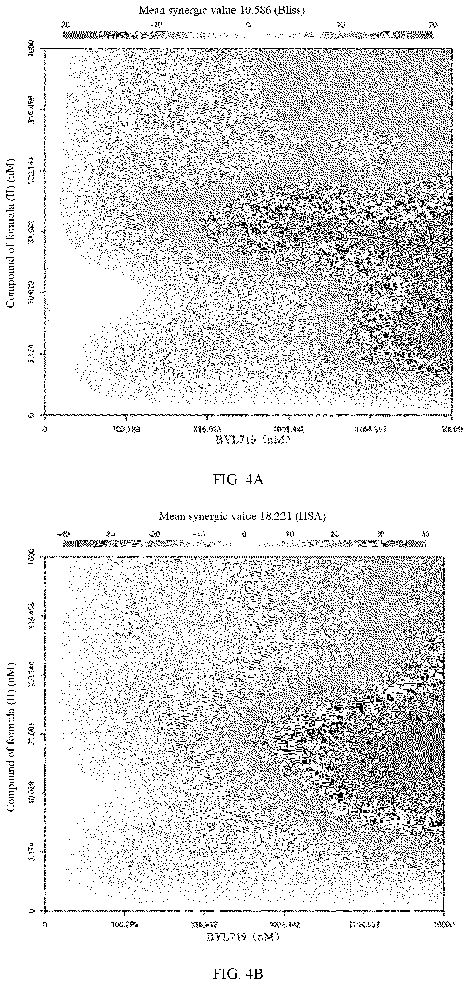

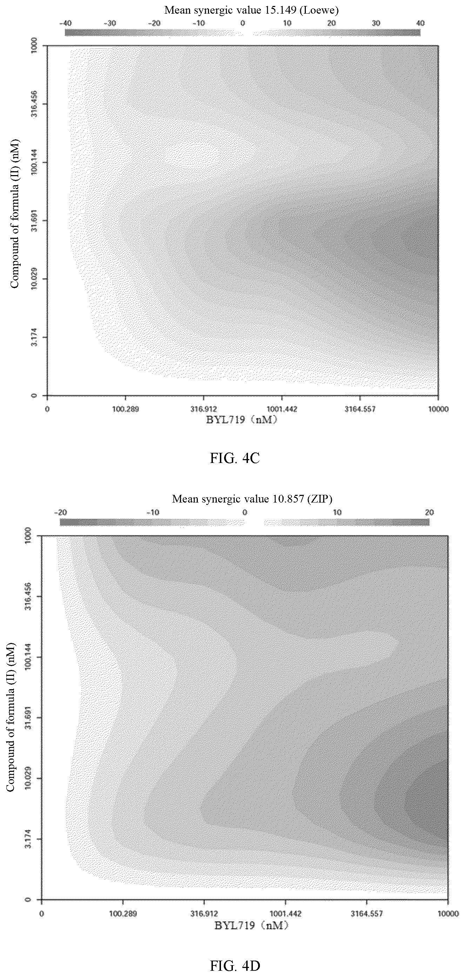

The combination effect is shown in FIG. 4. In human rectal adenocarcinoma cells SW837, 3.17 nM-1000 nM of the compound of formula (II) was combined with 100 nM-10000 nM of BYL719. The mean synergic values calculated using the four statistical models (Bliss (FIG. 4A), HSA (FIG. 4B), Loewe (FIG. 4C), and ZIP (FIG. 4D)) were 10.586, 18.221, 15.149, and 10.857, respectively, all of which were greater than 5. The results show that the combination of the compound of formula (II) with BYL719 has a very good synergic effect in tumor cells.

### Example 3. Effect of combination of compound of formula (II) with GDC0941 (pictilisib) on tumor cells LU99

Human non-small cell lung cancer cells LU99 were plated into a 384-well plate at 600 cells/well. After the cells adhered to the well wall (24 h), the serially diluted compound of formula (II) and GDC0941 were added in duplicate for each concentration. After 120 h, the cell viability was measured by Cell Titer-Glo method. The Cell Titer-Glo reagent was added at 30 µL/well. The plate was shaken for 2 min and left to stand for 10 min. The RLU value was read on the Envision system.

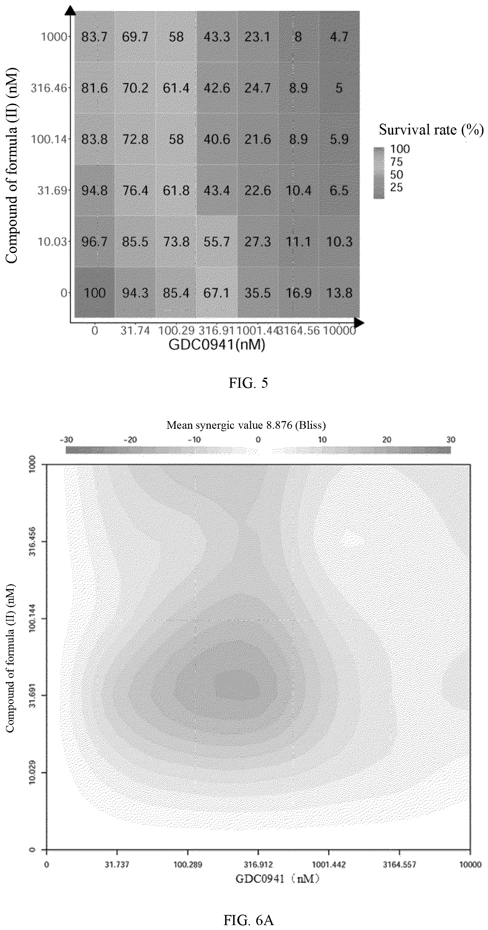

The cell survival rate calculation results are shown in FIG. 5. In human non-small cell lung cancer cells LU99, for example, the survival rate of cells receiving 100 nM of the compound of formula (II) alone was 83.8%, the survival rate of cells receiving 100 nM of GDC0941 alone was 85.4%, and the survival rate of cells receiving the combination of 100 nM of the compound of formula (II) with 100 nM of GDC0941 was 58%.

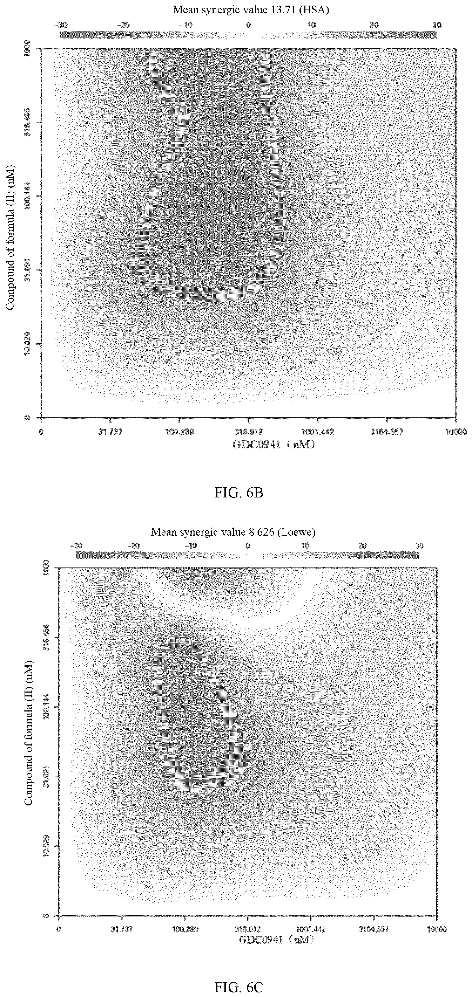

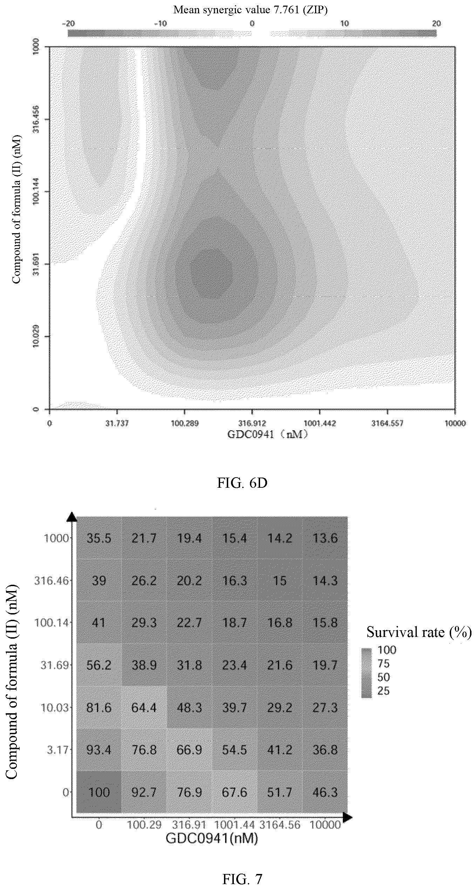

The combination effect is shown in FIG. 6. In human non-small cell lung cancer cells LU99, 10 nM-1000 nM of the compound of formula (II) was combined with 31.74 nM-10000 nM of GDC0941. The mean synergic values calculated using the four statistical models (Bliss (FIG. 6A), HSA (FIG. 6B), Loewe (FIG. 6C), and ZIP (FIG. 6D)) were 8.876, 13.71, 8.626, and 7.761, respectively, all of which were greater than 5. The results show that the combination of the compound of formula (II) with GDC0941 has a very good synergic effect in tumor cells.

### Example 4. Effect of combination of compound of formula (II) with GDC0941 on tumor cells SW837

Human rectal adenocarcinoma cells SW837 were plated into a 384-well plate at 800 cells/well. After the cells adhered to the well wall (24 h), the serially diluted compound of formula (II) and GDC0941 were added in duplicate for each concentration. After 120 h, the cell viability was measured by Cell Titer-Glo method. The Cell Titer-Glo reagent was added at 30 µL/well. The plate was shaken for 2 min and left to stand for 10 min. The RLU value was read on the Envision system.

The cell survival rate calculation results are shown in FIG. 7. In human rectal adenocarcinoma cells SW837, for example, the survival rate of cells receiving 31.69 nM of the compound of formula (II) alone was 56.2%, the survival rate of cells receiving 1000 nM of GDC0941 alone was 67.6%, and the survival rate of cells receiving the combination of 31.69 nM of the compound of formula (II) with 1000 nM of GDC0941 was 23.4%.

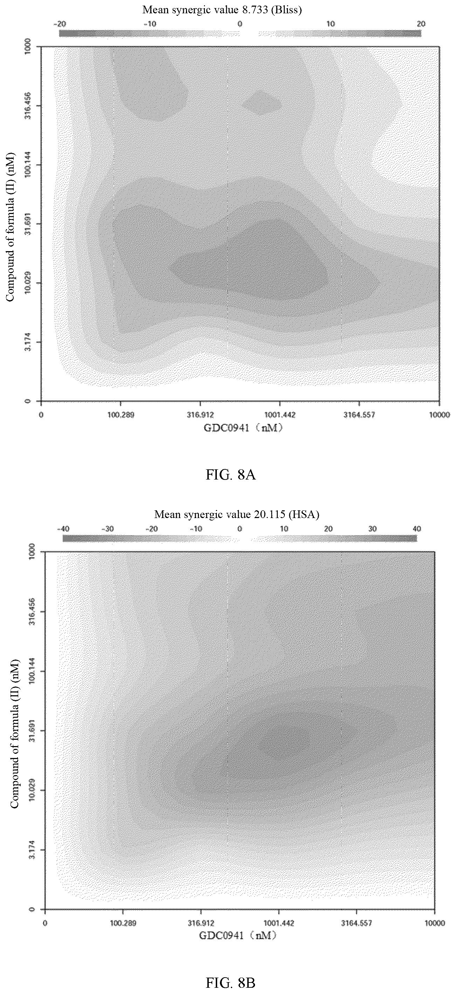

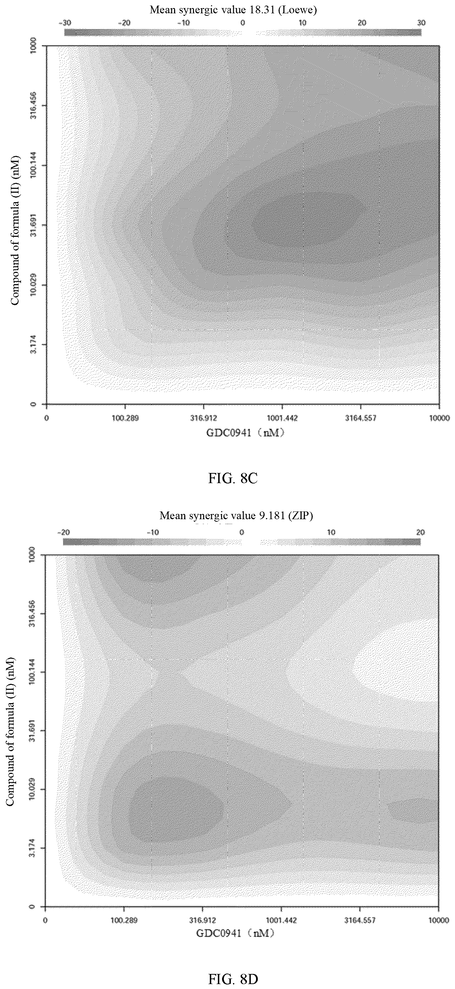

The combination effect is shown in FIG. 8. In human rectal adenocarcinoma cells SW837, 3.17 nM-1000 nM of the compound of formula (II) was combined with 100 nM-10000 nM of GDC0941. The mean synergic values calculated using the four statistical models (Bliss (FIG. 8A), HSA (FIG. 8B), Loewe (FIG. 8C), and ZIP (FIG. 8D)) were 8.733, 20.115, 18.31, and 9.181, respectively, all of which were greater than 5. The results show that the combination of the compound of formula (II) with GDC0941 has a very good synergic effect in tumor cells.

### Example 5. Effect of combination of compound of formula (II) with GDC0941 on tumor cells SW1463

Human rectal adenocarcinoma cells SW1463 were plated into a 384-well plate at 1200 cells/well. After the cells adhered to the well wall (24 h), the serially diluted compound of formula (II) and GDC0941 were added in duplicate for each concentration. After 120 h, the cell viability was measured by Cell Titer-Glo method. The Cell Titer-Glo reagent was added at 30 µL/well. The plate was shaken for 2 min and left to stand for 10 min. The RLU value was read on the Envision system.

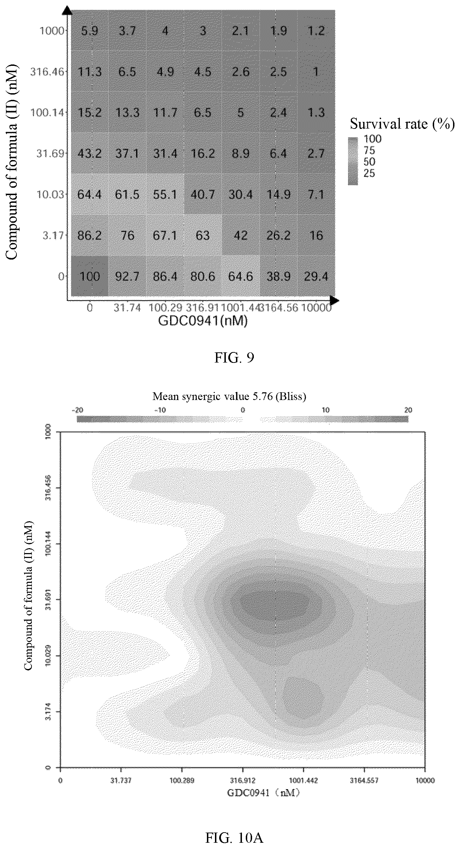

The cell survival rate calculation results are shown in FIG. 9. In human rectal adenocarcinoma cells SW1463, for example, the survival rate of cells receiving 31.69 nM of the compound of formula (II) alone was 43.2%, the survival rate of cells receiving 1000 nM of GDC0941 alone was 64.6%, and the survival rate of cells receiving the combination of 31.69 nM of the compound of formula (II) with 1000 nM of GDC0941 was 8.9%.

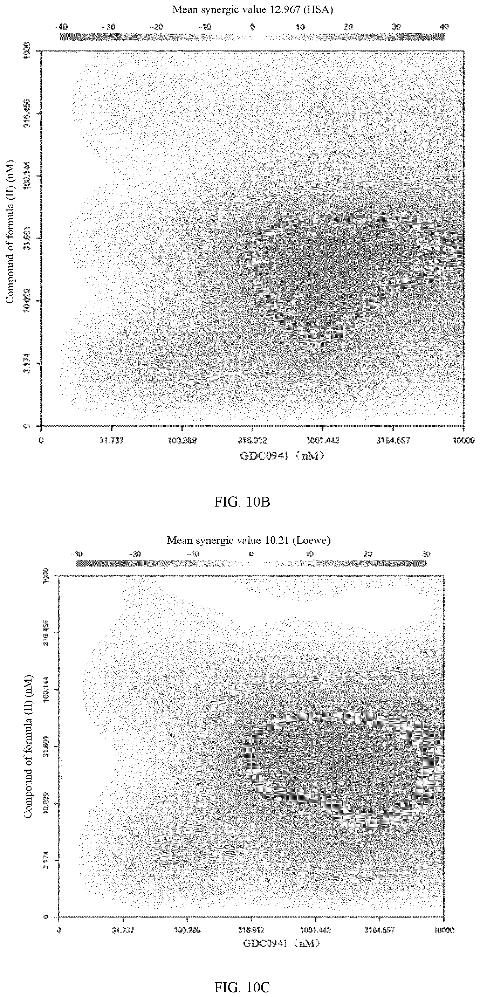

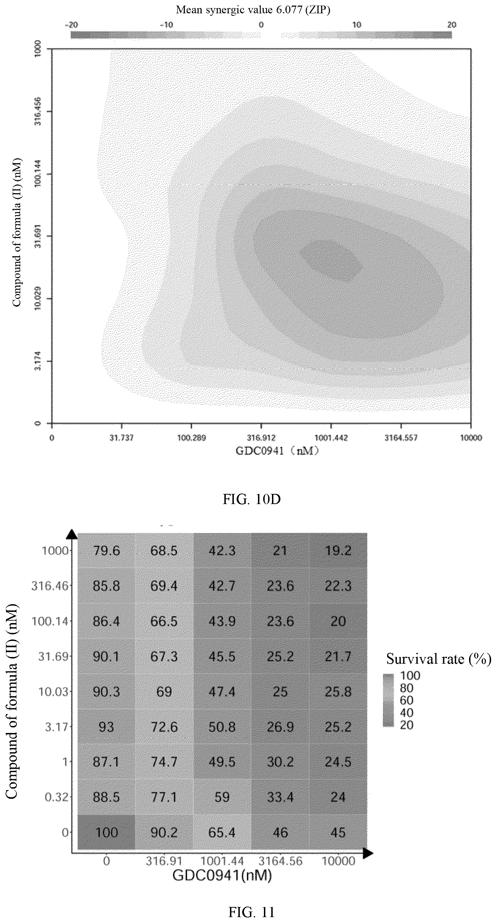

The combination effect is shown in FIG. 10. In human rectal adenocarcinoma cells SW1463, 3.17 nM-1000 nM of the compound of formula (II) was combined with 31.74 nM-10000 nM of GDC0941. The mean synergic values calculated using the four statistical models (Bliss (FIG. 10A), HSA (FIG. 10B), Loewe (FIG. 10C), and ZIP (FIG. 10D)) were 5.76, 12.967, 10.21, and 6.077, respectively, all of which were greater than 5. The results show that the combination of the compound of formula (II) with GDC0941 has a very good synergic effect in tumor cells.

### Example 6. Effect of combination of compound of formula (II) with GDC0941 on tumor cells SW1573

Human non-small cell lung cancer cells SW1573 were plated into a 384-well plate at 600 cells/well. After the cells adhered to the well wall (24 h), the serially diluted compound of formula (II) and GDC0941 were added in duplicate for each concentration. After 120 h, the cell viability was measured by Cell Titer-Glo method. The Cell Titer-Glo reagent was added at 30 µL/well. The plate was shaken for 2 min and left to stand for 10 min. The RLU value was read on the Envision system.

The cell survival rate calculation results are shown in FIG. 11. In human non-small cell lung cancer cells SW1573, for example, the survival rate of cells receiving 100 nM of the compound of formula (II) alone was 86.4%, the survival rate of cells receiving 3164 nM of GDC0941 alone was 46%, and the survival rate of cells receiving the combination of 100 nM of the compound of formula (II) with 3164 nM of GDC0941 was 23.6%.

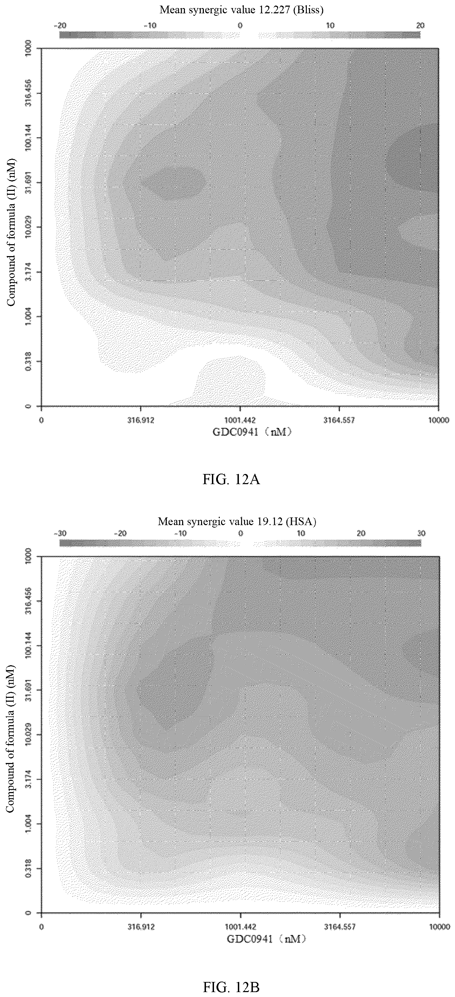

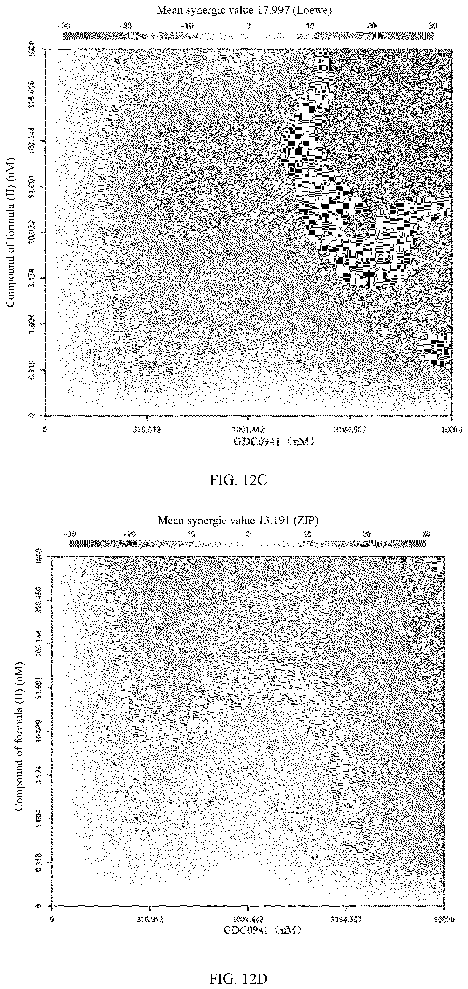

The combination effect is shown in FIG. 12. In human non-small cell lung cancer cells SW1573, 0.32 nM-1000 nM of the compound of formula (II) was combined with 316.91 nM-10000 nM of GDC0941. The mean synergic values calculated using the four statistical models (Bliss (FIG. 12A), HSA (FIG. 12B), Loewe (FIG. 12C), and ZIP (FIG. 12D)) were 12.227, 19.12, 17.997, and 13.191, respectively, all of which were greater than 5. The results show that the combination of the compound of formula (II) with GDC0941 has a very good synergic effect in tumor cells.

### Example 7. Effect of combination of compound of formula (II) with SY-5609 (Synonyms) on tumor cells NCI-H2122

Human non-small cell lung cancer cells NCI-H2122 were plated into a 384-well plate at 600 cells/well. After the cells adhered to the well wall (24 h), the serially diluted compound of formula (II) and SY-5609 were added in duplicate for each concentration. After 120 h, the cell viability was measured by Cell Titer-Glo method. The Cell Titer-Glo reagent was added at 30 µL/well. The plate was shaken for 2 min and left to stand for 10 min. The RLU value was read on the Envision system.

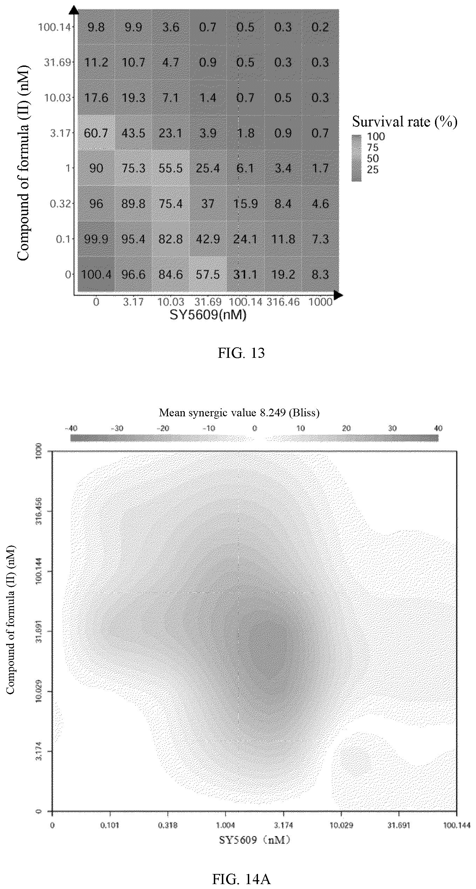

The cell survival rate calculation results are shown in FIG. 13. In human non-small cell lung cancer cells NC-H2122, for example, the survival rate of cells receiving 3.17 nM of the compound of formula (II) alone was 60.7%, the survival rate of cells receiving 31.69 nM of SY-5609 alone was 57.5%, and the survival rate of cells receiving the combination of 3.17 nM of the compound of formula (II) with 31.69 nM of SY-5609 was 3.9%.

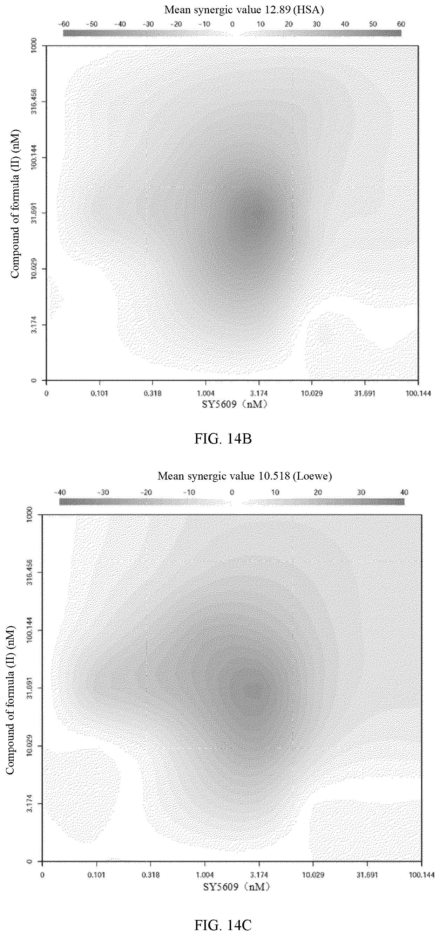

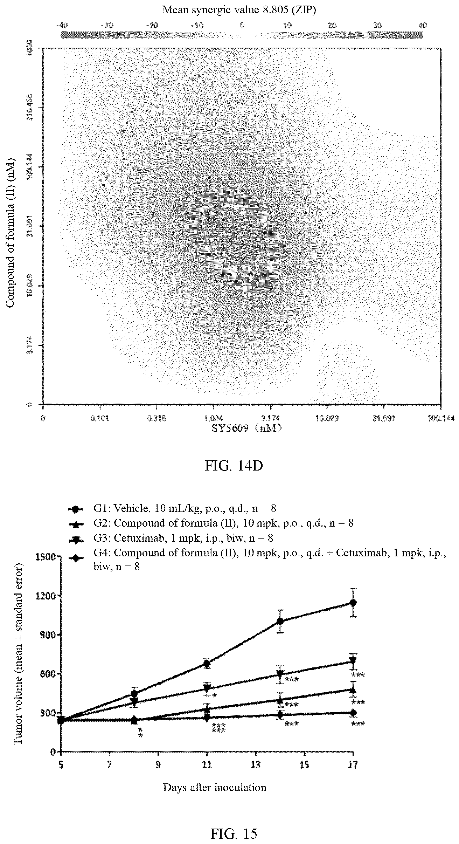

The combination effect is shown in FIG. 14. In human non-small cell lung cancer cells NC-H2122, 0.1 nM-100 nM of the compound of formula (II) was combined with 3.17 nM-1000 nM of SY-5609. The mean synergic values calculated using the four statistical models (Bliss (FIG. 14A), HSA (FIG. 14B), Loewe (FIG. 14C), and ZIP (FIG. 14D)) were 8.249, 12.89, 10.518, and 8.805, respectively, all of which were greater than 5. The results show that the combination of the compound of formula (II) with SY-5609 has a very good synergic effect in tumor cells.

### Example 8. In vivo anti-tumor pharmacological evaluation of combination of compound of formula (II) with cetuximab in NCI-H2122 human non-small cell lung cancer model

### Materials and reagents:

NCI-H2122 (ATCC, CRL-5807), penicillin-streptomycin (Gibco, 15140-122), RPMI1640 (Gibco, 11875-093), Trypsin-EDTA (Gibco, 25200-072), FBS (Gibco, 10099-141C), Solutol HS 15 (Sigma, 42966-1kg), HP-β-CD (Accela, SY004863 100 g).

During the study, the health of the animals was observed daily. If there was a 10% weight loss in the animals, the administration dose was halved; if there was a 15% weight loss, the was discontinued until a normal body weight was restored; or if the tumor volume in the animals exceeded 2,000 mm³, the mouse was euthanized immediately. If the health condition met the following criteria, euthanasia was performed by the veterinarian:
Significant emaciation, or a weight loss greater than 20%.
incapability for *ad libitum* food and water intake.
Infections, severe tumor ulcerations, or hemangiomas.

The occurrence and continuous worsening of the following clinical symptoms: piloerection, hunched back, paleness of the ears, nose, eyes or paws, shortness of breath, twitching, diarrhea, pain, dehydration, or near-death.

### Animals:

BALB/c nude mice, aged 6-8 weeks, female, purchased from Beijing Vital River Laboratory Animal Technology Co., Ltd., Certificate No. 20170011008676, and housed in an SPF-grade environment.

### Test compound:

Cetuximab, purchased from MERCK, Lot No. G00XED.

Preparation: the compound of the formula (II) was prepared in a vehicle of 5% DMSO + 10% Solutol HS 15 + 85% (6% HP-β-CD); cetuximab was prepared in a 0.9% sodium chloride injection.

### Methodology and procedures

NCI-H2122 cells were cultured in an RPMI 1640 medium containing 10% of FBS and 1% of penicillin-streptomycin in an incubator at 37 °C and 5% CO2. The cells were digested with 0.25% trypsin-EDTA for passaging as per conventional practice. When the cells were in the logarithmic growth phase and the confluence reached 80%-0%, the cells were collected and counted. The cells were collected and adjusted to a final concentration of 2.0 × 106 cells/mL. Mice were grafted subcutaneously at the right dorsal region in a volume of 0.15 mL/3 × 105 cells/mouse. When the tumors grew to an average size of about 242 mm3 (D5 after grafting), 32 tumor-bearing mice were selected, randomized into 4 groups of 8 mice each as per the tumor volume and the body weight, and treated according to the administration regimen shown in Table 1. The dose in mice was 10 mL/kg. The mice were weighed on an electronic balance twice weekly and the tumor volume was measured with a vernier caliper twice weekly. The study was ended on D17 after grafting, and the mice were euthanized after tumor volume measurement.

**Table 1. Administration regimen of therapeutic agents**

| Group | Therapeutic agent | Number of animals | Administration route | Dose (mpk) | Administration frequency | Treatment cycle (days) |
|---|---|---|---|---|---|---|
| G1 | Vehicle | 8 | p.o | -- | q.d | 12 |
| G2 | Compound of formula (II) | 8 | p.o | 10 | q.d | 12 |
| G3 | Cetuximab | 8 | i.p. | 1 | biw | 12 |
| G4 | Compound of formula (II) + cetuximab | 8 | Compound of formula (II) p.o. + cetuximab i.p. | Compound of formula (II) 10 mpk + Cetuximab 1 mpk | Compound of formula (II) q.d. Cetuximab biw | 12 |

| | | | | | | |
|---|---|---|---|---|---|---|
| Note: p.o., denotes oral administration; i.p., denotes intraperitoneal injection; q.d., denotes once daily; biw denotes twice weekly. | | | | | | |

The main evaluation indexes were as follows:
Tumor volume (TV) = (L × W²)/2, where L denotes the long diameter of the tumor, and W is the short diameter of the tumor.

Tumor growth inhibition (TGI):
TGI (%) = [1 - (avTᵢ₋₀/avCᵢ₋₀)] × 100%, where avTᵢ₋₀ denotes the average tumor volume of the treatment group on a specific day minus the average tumor volume of the treatment group at the baseline; avCᵢ₋₀ denotes the average tumor volume of the vehicle control group on a specific day minus the average tumor volume of the vehicle control group at the baseline.

Tumor weight inhibition (TWI):
TWI = (1 - TW_{treatment/Dx}/TW_{control/Dx}) × 100%, where TW_{control} denotes the average tumor weight (g) of the control group, and TWₜᵣₑₐₜₘₑₙₜ denotes the average tumor weight (g) of the treatment group.

Relative change of body weight (RCBW):
RCBW (%) = (BWᵢ - BW₀)BW₀ × 100%, where BWᵢ is the body weight of a mouse on a specific day and BW0 is the body weight of the mouse at the baseline.

### Data analysis, results, and conclusions

All experimental data analysis and graphing were performed using GraphPad Prism software (GraphPad Software). Tumor volumes and the body weights of the animals in all groups were statistically compared using two-way ANOVA, Dunnett's multiple comparisons test. Tumor weights of the groups at the endpoint were statistically compared using one-way ANOVA, Dunnett's multiple comparisons test. P < 0.05 denotes a statistically significant difference. The tumor volume and weight and body weight of animals are expressed by mean ± standard error (SEM). The results are shown in Table 2 below.

**Table 2. Results**

| Group | Tumor volume (mm³) | | TGI (%) | Tumor weight | TWI (%) | Body weight | | RCBW (%) |
|---|---|---|---|---|---|---|---|---|
| | After grafting Day 5 | After grafting Day17 | | After grafting Day 17 | | After grafting Day 5 (g) | After grafting Day 17 (g) | |
| G1 | 242.15±11.60 | 1144.11±107.53 | -- | 0.60±0.05 | -- | 21.12±0.31 | 18.60±0.38 | -11.95±0.63 |
| G2 | 242.93±11.14 | 479.14±58.84*** | 73.81 | 0.30±0.04*** | 49.37 | 21.16±0.47 | 20.97±0.38 | -0.78±1.22*** |
| G3 | 242.79±10.09 | 692.74±62.50*** | 50.11 | 0.52±0.05 | 13.24 | 21.11±0.24 | 19.74±0.25 | -6.46±1.28*** |
| G4 | 243.76±10.32 | 300.31±32.99*** | 93.73 | 0.20±0.02*** | 66.60 | 20.63±0.31 | 20.68±0.44 | 0.43±2.77*** |

| | | | | | | | | |
|---|---|---|---|---|---|---|---|---|
| Note: ****p* < 0.001, as compared with the G1 vehicle control group separately. | | | | | | | | |

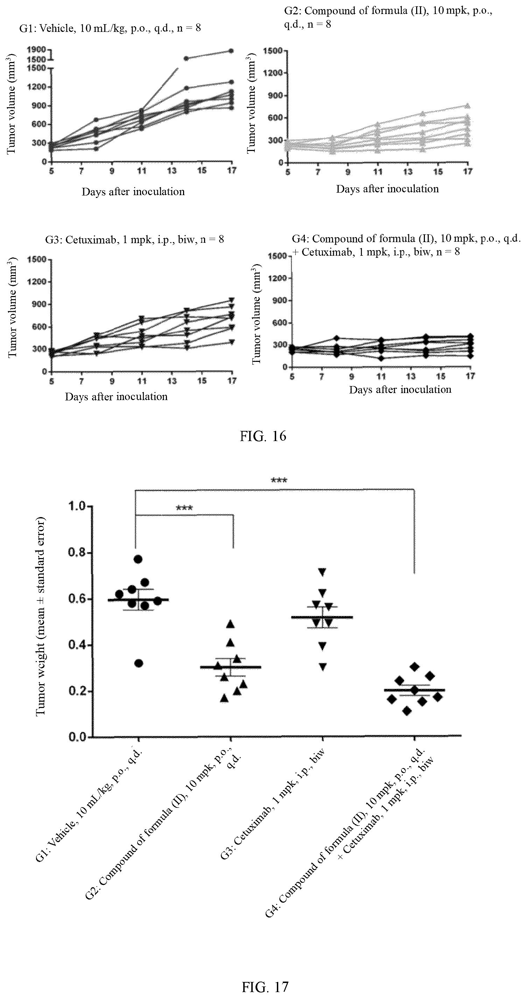

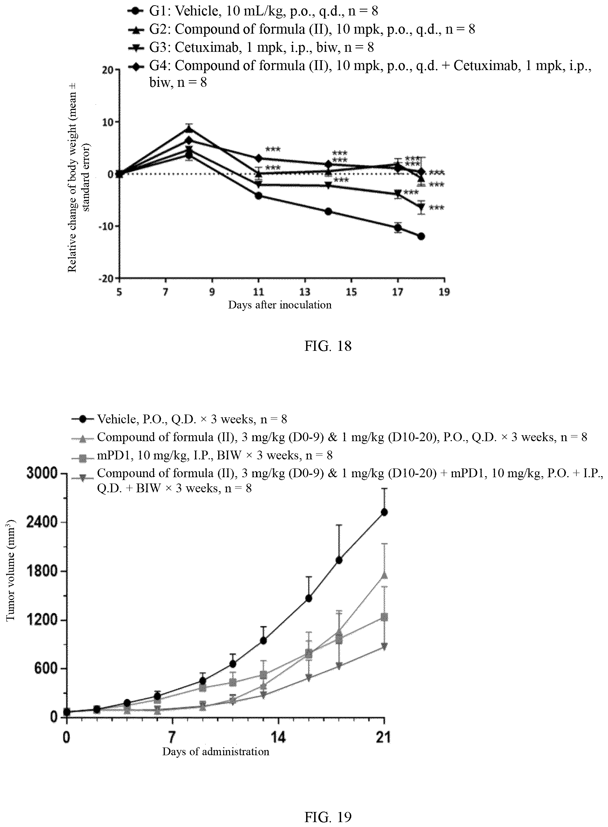

The effects on tumor growth in NCI-H2122 tumor-bearing mice in all treatment groups are shown in FIGs. 15 and 16. The tumor weights in the tumor-bearing mice in all treatment groups at endpoint are shown in FIG. 17. At the endpoint, animals were euthanized and dissected, and tumor tissues were collected, weighed, and photographed. The relative changes of body weight in the tumor-bearing mice in all groups are shown in FIG. 18.

The results show that in the NCI-H2122 human non-small cell lung cancer model, the combination of the compound of the formula (II) administered orally once daily and cetuximab intraperitoneally injected twice weekly can significantly inhibit the growth of NCI-H2122 tumors, while exhibiting a certain synergic effect. Compared with the weight loss in the vehicle control group, there was no significant difference in body weight loss of the animals in the groups treated with the compound of formula (II) at 10 mpk, cetuximab at 1 mpk, and the combination of the compound of formula (II) at 10 mpk with cetuximab at 1 mpk. These differences were statistically significant.

### Example 9. In vivo anti-tumor pharmacological evaluation of combination of compound of formula (II) with mPD1 in mouse CT-26 cell model

The animals and the main evaluation indexes were the same as those of Example 8.

### Test compound:

Mouse PD1 antibody (mPD1), purchased from BioXcell-BE0146, Lot No. 810421D1.

Preparation: the compound of the formula (II) was prepared in a vehicle of 5% DMSO + 10% Solutol HS 15 + 85% (6% HP-β-CD); mPD1 was prepared in PBS.

### Methodology and procedures

The mice were grafted subcutaneously at the right dorsal region with CT26 KRASG12C KI cells (CT26 cells carrying KRASG12C mutation) in a volume of 0.1 mL/mouse. On day 12 after cell inoculation (D0), the grouping and treatment were started after the mean tumor volume reached 69 mm³. 32 tumor-bearing mice were selected, randomized into 4 groups of 8 mice each as per the tumor volume and the body weight, and treated according to the administration regimen shown in Table 3. The administration volume in mice was 10 mL/kg. The mice were weighed on an electronic balance twice weekly and the tumor volume was measured with a vernier caliper twice weekly.

**Table 3. Administration regimen of therapeutic agents**

| Group | Therapeutic agent | Number of animals | Administration route | Dose (mpk) | Administration frequency | Treatment cycle (days) |
|---|---|---|---|---|---|---|
| G1 | Vehicle | 8 | p.o | -- | q.d | 21 |
| G2 | Compound of formula (II) | 8 | p.o | 3mpk (DO-9)&lmpk (D10-20) | q.d | 21 |
| G3 | mPD1 | 8 | i.p. | 10 | biw | 21 |
| G4 | Compound of formula (II) + mPD1 | 8 | Compound of formula (II) p.o. + mPD1 i.p. | Compound of formula (II) 3 mpk (D0-9) & 1 mpk (D10-20) + mPD1 10 mpk | Compound of formula (II) q.d. mPD1 biw | 21 |

| | | | | | | |
|---|---|---|---|---|---|---|
| Note: p.o., denotes oral administration; i.p., denotes intraperitoneal injection; q.d., denotes once daily; biw denotes twice weekly. | | | | | | |

The main evaluation indexes were tumor volume, TGI, TWI, and RCBW. The calculation formula and data analysis were the same as in Example 8, and the results are shown in Table 4 below:

**Table 4. Results**

| Group | Tumor volume (mm³) | | TGI (%) | Tumor weight | TWI (%) | Body weight | | RCBW (%) |
|---|---|---|---|---|---|---|---|---|
| | After grafting Day 12 | Day 33 after grafting | | After grafting Day 33 | | Day 12 after grafting (g) | After grafting Day 33 (g) | |
| G1 | 69.13 ±6.81 | 1939.38 ±426.98 | -- | 2.86 ±1.09 | -- | 21.27 ±0.57 | 23.03 ±0.80 | 8.20 ±1.94 |
| G2 | 68.75 ±6.65 | 1065.38 ±251.12 | 46.74 | 1.84 ±1.12 | 35.38 | 21.29 ±0.29 | 22.76 ±0.37 | 6.93 ±1.42 |
| G3 | 68.88 ±6.59 | 968.13 ±314.32 | 51.94 | 1.24 ±1.07* | 56.71 | 21.51 ±0.49 | 22.70 ±0.49 | 5.69 ±1.87 |
| G4 | 68.75 ±6.56 | 631.50 ±283.15* | 69.92 | 0.90 ±1.79** | 68.52 | 21.32 ±0.54 | 22.52 ±0.72 | 5.56 ±1.59 |

| | | | | | | | | |
|---|---|---|---|---|---|---|---|---|
| Note: **p* < 0.05, ***p* < 0.01, as compared with the G1 vehicle control group separately. | | | | | | | | |

The results show that in the mouse CT26 KRASG12C KI cell model, the compound of formula (II) and the mouse PD1 antibody (mPD1) can significantly inhibit the tumor growth, while exhibiting a certain synergic effect.

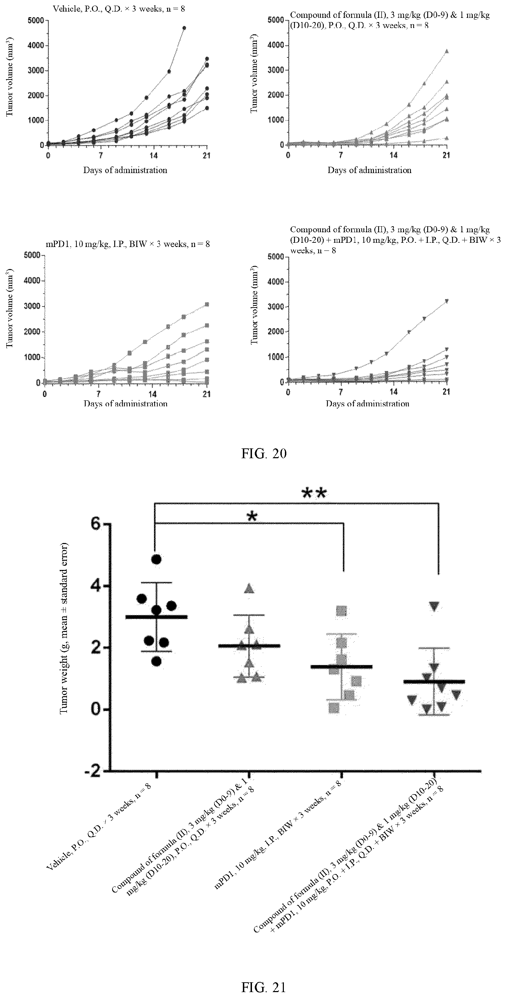

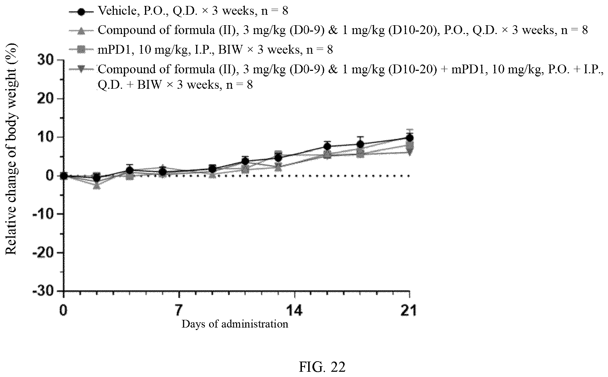

The effects on tumor growth in CT26 KRAS G12C KI tumor-bearing mice in all treatment groups are shown in FIGs. 19 and 20. The tumor weights in the tumor-bearing mice in all treatment groups at endpoint are shown in FIG. 21. At the endpoint, animals were euthanized and dissected, and tumor tissues were collected and weighed. The relative changes of body weight in the tumor-bearing mice in all groups are shown in FIG. 22.

The results show that in the mouse colon cancer CT26 KRAS G12C KI cell subcutaneous graft tumor model, both the compound of formula (II) monotherapy group and the mPD1 monotherapy group showed mild tumor inhibitory effects, with the mean tumor volumes being 1,065 mm³ (T/C = 54.92%; TGI = 46.74%, p = 0.1640) and 968 mm³ (T/C = 49.91%; TGI = 51.94%, p = 0.1094), respectively. The combination treatment group of the compound of formula (II) with mPD1 exhibited a significant tumor inhibitory effect, with a mean tumor volume of 632 mm³ (T/C = 32.56%; TGI = 69.92%, p = 0.0223). At the end of study, one mouse in the group had a tumor volume of less than 50 mm³, which was considered complete regression. During the treatment, the tumor-bearing mice showed good tolerance to the test compounds, and none of the mice in any treatment group exhibited significant weight loss or death due to the treatment.

It can be seen from the above results that the KRAS inhibitor of formula (I), when used in combination with PI3K inhibitors represented by BYL719 and GDC0941, EGFR antibodies represented by cetuximab, CDK7 inhibitors represented by SY-5609, and PD-1 antibodies represented by mPD1, may have significant synergistic effects in treating lung adenocarcinoma, rectal adenocarcinoma, lung cancer, or the like.

The aforementioned description of the specific exemplary embodiments of the present invention has been presented for the purposes of illustration and description. It is not intended to limit the present invention to the precise forms disclosed, and it is apparent that many modifications and variations are possible according to the teachings above. The exemplary embodiments are chosen and described for the purposes of explaining specific principles of the present invention and practical application to enable those skilled in the art to implement and adopt various exemplary embodiments of the present invention and various options and modifications. The protection scope of the present invention is defined by the claims and equivalents thereof.

## Claims

1. A pharmaceutical composition or kit, comprising a therapeutically effective amount of a KRAS inhibitor and a therapeutically effective amount of at least one additional anti-cancer therapeutic agent, as well as a pharmaceutically acceptable carrier;
wherein the KRAS inhibitor comprises the compound of formula (I), or a pharmaceutically acceptable salt, a stereoisomer, a solvate, or a prodrug thereof; wherein R is CH₃ or CD₃;

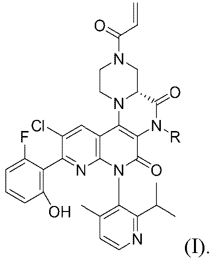

2. The pharmaceutical composition or kit according to claim 1, wherein the additional anti-cancer therapeutic agent is selected from a PD-1 pathway inhibitor, a PD-L1 pathway inhibitor, an EGFR-targeting agent, a CDK inhibitor, and a PI3K inhibitor;
preferably, the PD-1 pathway inhibitor is selected from a PD-1 antagonist, a PD-1-binding antagonist, a small molecule PD-1 antagonist, a PD-1 inhibitor, and an anti-PD-1 bioproduct;
preferably, the PD-L1 pathway inhibitor is selected from a PD-L1 antagonist, a PD-L1-binding antagonist, a small molecule PD-L1 antagonist, a PD-L1 inhibitor, and an anti-PD-L1 bioproduct;
preferably, the EGFR-targeting agent is selected from an EGFR inhibitor and an EGFR antibody or an antigen-binding fragment thereof;
preferably, the CDK inhibitor is selected from a CDK4/6 inhibitor, a CDK2/4/6 inhibitor, a CDK7 inhibitor, a CDK8 inhibitor, a CDK9 inhibitor, a CDK10 inhibitor, and a CDK11 inhibitor;
preferably, the PI3K inhibitor is selected from a PI3Kγ inhibitor, a PI3Kδ inhibitor, a PI3Kβ inhibitor, a PI3Kα inhibitor, and a pan-PI3K inhibitor.

3. The pharmaceutical composition or kit according to claim 1, wherein the additional anti-cancer therapeutic agent is selected from a PD-1 inhibitor, a PD-L1 inhibitor, an EGFR inhibitor, an EGFR antibody or an antigen-binding fragment thereof, a CDK7 inhibitor, and a PI3K inhibitor;
preferably, the PD-1 inhibitor is selected from AMG404, pembrolizumab, nivolumab, pembrolizumab, pidilizumab, cemiplimab, tislelizumab, spartalizumab, RN888, mAb15, MEDI-0680, BGB-108, spartalizumab, IBI-308, mDX-400, SHR-1210, PF-06801591, PDR-001, GB-226 and STI-1110, and a biosimilar, a biobetter and a bioequivalent of the inhibitors;
preferably, the PD-L1 inhibitor is selected from BMS-936559, AMP-714, atezolizumab, durvalumab, avelumab, avelumab, ALN-PDL, TSR-042, KD-033, CA-170, STI-1014 and KY-1003, and a biosimilar, a biobetter and a bioequivalent of the inhibitors;
preferably, the EGFR inhibitor is selected from afatinib, erlotinib, lapatinib, gefitinib, osimertinib, icotinib, pyrotinib, and olmutinib;
preferably, the EGFR antibody or the antigen-binding fragment thereof is selected from cetuximab, nimotuzumab, panitumumab, zalutumumab, matuzumab, and necitumumab, more preferably, cetuximab; preferably, the CDK7 inhibitor is selected from SY-5609, SY-1365, CT-7001, and BTX-A51, more preferably, SY-5609;
preferably, the PI3K inhibitor is selected from BYL719, GDC0941, AMG511, LY294002, BEZ235, BKM120, and GSK-2636771, more preferably, BYL719 and GDC0941.

4. The pharmaceutical composition or kit according to claim 1, wherein the compound of formula (I) is selected from the following group:

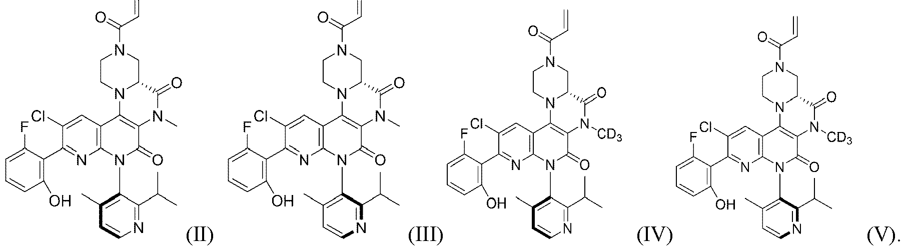

5. The pharmaceutical composition or kit according to claim 1 or 2, wherein the KRAS inhibitor is the compound of formula (II) or a pharmaceutically acceptable salt thereof; 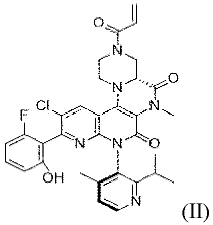
preferably, the pharmaceutical composition or kit comprises the compound of formula (II) and the PD-1 pathway inhibitor;
preferably, the pharmaceutical composition or kit comprises the compound of formula (II) and the PD-L1 pathway inhibitor;
preferably, the pharmaceutical composition or kit comprises the compound of formula (II) and the EGFR-targeting agent, more preferably, the compound of formula (II) and cetuximab;
preferably, the pharmaceutical composition or kit comprises the compound of formula (II) and the CDK7 inhibitor, more preferably, the compound of formula (II) and SY-5609;
preferably, the pharmaceutical composition or kit comprises the compound of formula (II) and the PI3K inhibitor, more preferably, the compound of formula (II) and BYL719, or the compound of formula (II) and GDC0941.

6. The pharmaceutical composition or kit according to claim 5, comprising 0.1-1000 nM of the KRAS inhibitor and 1-10000 nM of the additional anti-cancer therapeutic agent, wherein
preferably, the pharmaceutical composition or kit comprises 10 mg/mL of the compound of formula (II) and 1 mg/mL of cetuximab;
preferably, the pharmaceutical composition or kit comprises 1-3 mg/kg of the compound of formula (II) and 10 mg/kg of the PD-1 antibody;
preferably, the pharmaceutical composition or kit comprises a pharmaceutical dose equivalent to 10 mpk of the compound of formula (II) and 1 mpk of cetuximab;
preferably, the pharmaceutical composition or kit comprises a pharmaceutical dose equivalent to 1-3 mpk of the compound of formula (II) and 10 mpk of the PD-1 antibody;
preferably, the pharmaceutical composition or kit comprises 0.1-100 nM of the compound of formula (II) and 3.17-1000 nM of SY-5609;
preferably, the pharmaceutical composition or kit comprises 1-1000 nM of the compound of formula (II) and 100-10000 nM of BYL719, 3-1000 nM of the compound of formula (II) and 100-10000 nM of BYL719, or 10-1000 nM of the compound of formula (II) and 100-10000 nM of BYL719;
preferably, the pharmaceutical composition or kit comprises 0.1-1000 nM of the compound of formula (II) and 10-10000 nM of GDC0941, 3.17-1000 nM of the compound of formula (II) and 31.74-10000 nM of GDC0941, 3.17-1000 nM of the compound of formula (II) and 100-10000 nM of GDC0941, 10-1000 nM of the compound of formula (II) and 31.74-10000 nM of GDC0941, or 0.32-1000 nM of the compound of formula (II) and 316.91-10000 nM of GDC0941.

7. A method for treating a cancer, comprising: administering to a subject in need the KRAS inhibitor and the additional anti-cancer therapeutic agent described in claim 1, wherein a therapeutically effective amount of the KRAS inhibitor and a therapeutically effective amount of the additional anti-cancer therapeutic agents may be administered simultaneously, separately, or sequentially;
preferably, the method further comprises: administering to the subject an additional therapy selected from one or more of a radiation therapy, a surgery, a chemotherapy, a gene therapy, a DNA therapy, a viral therapy, an RNA therapy, an immunotherapy, bone marrow transplantation, a nano-therapy, a monoclonal antibody therapy, and a phototherapy; the additional therapy may be in the form of an adjuvant therapy or a neoadjuvant therapy.

8. Use of the pharmaceutical composition or kit according to any one of claims 1-6 in preparing a medicament for treating a cancer, wherein a therapeutically effective amount of the KRAS inhibitor and a therapeutically effective amount of the additional anti-cancer therapeutic agent may be administered simultaneously, separately, or sequentially;
preferably, the cancer is a solid tumor or a hematological tumor; preferably, the cancer is pancreatic ductal carcinoma, colorectal cancer, multiple myeloma, lung cancer, cutaneous melanoma, uterine corpus endometrioid carcinoma, uterine carcinosarcoma, thyroid cancer, acute myeloid leukemia, bladder urothelial carcinoma, gastric cancer, cervical cancer, head and neck squamous cell carcinoma, diffuse large B cell lymphoma, esophageal cancer, chronic lymphocytic leukemia, squamous cell lung cancer, small cell lung cancer, papillary renal cell carcinoma, adenoid cystic carcinoma, chromophobe renal cell carcinoma, liver cancer, invasive breast carcinoma, cervical squamous cell carcinoma, ovarian serous adenocarcinoma, adrenocortical carcinoma, prostate cancer, neuroblastoma, brain low-grade glioma, glioblastoma, medulloblastoma, esophageal squamous cell carcinoma, clear cell renal cell carcinoma, osteosarcoma, ovarian small cell carcinoma, rhabdomyoid tumor, sarcoma, small bowel neuroendocrine tumor, or T cell prolymphocytic leukemia;
preferably, the cancer is lung adenocarcinoma, colon cancer, rectal cancer, or lung cancer;
preferably, the cancer is lung adenocarcinoma, rectal adenocarcinoma, or lung cancer;
preferably, the lung cancer is small cell lung cancer or non-small cell lung cancer;
preferably, the cancer is a KRAS G12C mutant cancer.

9. Use of a therapeutically effective amount of a KRAS inhibitor and a therapeutically effective amount of at least one additional anti-cancer therapeutic agent in preparing a medicament, a pharmaceutical combination, or a kit for treating a cancer, wherein
the KRAS inhibitor is selected from one or more of the compound of formula (I) described in claim 1, the compounds of formula (II)-formula (V) described in claim 4, or a pharmaceutically acceptable salt, a stereoisomer, a solvate, or a prodrug thereof;
preferably, the additional anti-cancer therapeutic agent is selected from a PD-1 pathway inhibitor, a PD-L1 pathway inhibitor, an EGFR-targeting agent, a CDK inhibitor, and a PI3K inhibitor.

10. The use according to claim 9, wherein the PD-1 pathway inhibitor is selected from a PD-1 antagonist, a PD-1-binding antagonist, a small molecule PD-1 antagonist, a PD-1 inhibitor, and an anti-PD-1 bioproduct; preferably, the PD-L1 pathway inhibitor is selected from a PD-L1 antagonist, a PD-L1-binding antagonist, a small molecule PD-L1 antagonist, a PD-L1 inhibitor, and an anti-PD-L1 bioproduct;
preferably, the EGFR-targeting agent is selected from an EGFR inhibitor and an EGFR antibody or an antigen-binding fragment thereof;
preferably, the CDK inhibitor is selected from a CDK4/6 inhibitor, a CDK2/4/6 inhibitor, a CDK7 inhibitor, a CDK8 inhibitor, a CDK9 inhibitor, a CDK10 inhibitor, and a CDK11 inhibitor;
preferably, the PI3K inhibitor is selected from a PI3Kγ inhibitor, a PI3Kδ inhibitor, a PI3Kβ inhibitor, a PI3Kα inhibitor, and a pan-PI3K inhibitor.

11. The use according to claim 9, wherein the additional anti-cancer therapeutic agent is selected from a PD-1 inhibitor, a PD-L1 inhibitor, an EGFR inhibitor, an EGFR antibody or an antigen-binding fragment thereof, a CDK7 inhibitor, and a PI3K inhibitor;
preferably, the PD-1 inhibitor is selected from AMG404, pembrolizumab, nivolumab, pembrolizumab, pidilizumab, cemiplimab, tislelizumab, spartalizumab, RN888, mAb15, MEDI-0680, BGB-108, spartalizumab, IBI-308, mDX-400, SHR-1210, PF-06801591, PDR-001, GB-226 and STI-1110, and a biosimilar, a biobetter and a bioequivalent of the inhibitors;
preferably, the PD-L1 inhibitor is selected from BMS-936559, AMP-714, atezolizumab, durvalumab, avelumab, avelumab, ALN-PDL, TSR-042, KD-033, CA-170, STI-1014 and KY-1003, and a biosimilar, a biobetter and a bioequivalent of the inhibitors;
preferably, the EGFR inhibitor is selected from afatinib, erlotinib, lapatinib, gefitinib, osimertinib, icotinib, pyrotinib, and olmutinib;
preferably, the EGFR antibody or the antigen-binding fragment thereof is selected from cetuximab, nimotuzumab, panitumumab, zalutumumab, matuzumab, and necitumumab, more preferably, cetuximab; preferably, the CDK7 inhibitor is selected from SY-5609, SY-1365, CT-7001, and BTX-A51, more preferably, SY-5609;
preferably, the PI3K inhibitor is selected from BYL719, GDC0941, AMG511, LY294002, BEZ235, BKM120, and GSK-2636771, more preferably, BYL719 and GDC0941.
